# EUROPEAN PATENT SPECIFICATION

(11) **EP 3 959 335 B1**
(45) Date of publication and mention of the grant of the patent: **25.09.2024**
(21) Application number: 20721838.9
(22) Date of filing: 23.04.2020
(51) Int. Cl.: C12Q 1/6844

(54) **METHODS AND COMPOSITIONS FOR ISOTHERMAL DNA AMPLIFICATION**
VERFAHREN UND ZUSAMMENSETZUNGEN ZUR ISOTHERMISCHEN DNA-AMPLIFIKATION
PROCÉDÉS ET COMPOSITIONS POUR L'AMPLIFICATION ISOTHERMIQUE DU GÉNOME COMPLET

(30) Priority: 23.04.2019 NL 2022993
(43) Date of publication of application: 02.03.2022
(73) Proprietor: Synvolux IP B.V., 2333 CH Leiden (NL)
(72) Inventor: ZONDAG, Gerben Carolus Martinus, 2333 CH Leiden (NL); TEUNISSE, Bram Johannes, 2333 CH Leiden (NL); PAOLINI, Nahuel Alejandro, 2333 CH Leiden (NL)
(74) Representative: V.O.
(86) International application number: PCT/NL2020/050261
(87) International publication number: WO 2020/218924

(56) References cited:
- EP-A1- 2 206 789
- WO-A2-00/41524
- WO-A2-2017/172005
- HAFNER G J ET AL: "ISOTHERMAL AMPLIFICATION AND MULTIMERIZATION OF DNA BY BST DNA POLYMERASE", BIOTECHNIQUES RAPID DISPAT, INFORMA HEALTHCARE, US, vol. 30, no. 4, 1 April 2001 (2001-04-01), pages - 7, XP008011191, ISSN: 0736-6205

## Description

### FIELD:

The present invention is defined by the appended claims. Furthermore the application provides methods, compositions, and kits for replicating or amplifying native or denatured DNA template under isothermal conditions. More specifically, the invention is directed to the use of an RNA polymerase in methods for amplifying a DNA template without or with reduced exogenously-added oligonucleotide primers.

### 1 INTRODUCTION

Several techniques exist for amplification of DNA molecules. These include polymerase chain reaction (PCR), which involves repetitive rounds of reactions at different temperatures, and isothermal reactions which almost all involve a strand-displacing DNA polymerase. Examples of isothermal reactions include rolling circle amplification (RCA), multiple displacement amplification (MDA), loop-mediated isothermal amplification (LAMP), which involves specific primers to generate quasi circular DNA molecules, helicase-dependent amplification (HDA), and nicking enzyme amplification reaction (NEAR), which involve the generation of nicks in the DNA molecule that are used to prime replication.

Replication of a DNA molecule requires a free hydroxyl group that serves as a starting point for the addition of deoxyribonucleotides by a DNA polymerase. Typically, short, single stranded oligonucleotides or primers complementary to the template DNA molecule are exogenously added in order to prime replication of a DNA molecule by a DNA polymerase. Said single stranded primers may comprise a specific nucleotide sequence in order to amplify a specific DNA template, or comprise one or more generic and/or degenerate nucleotide sequences in order to amplify DNA templates in a semi-random manner. Said generic nucleotide sequences comprise, for example, random oligoribonucleotides, random hexameric and random nonameric DNA primers.

As an alternative, DNA replication may be initiated by a deoxyribonucleoside monophosphate that is covalently attached to a protein. Said proteins are used in nature by certain bacterial or animal viruses for genome replication in host cells (Salas, 1991. Annu. Rev. Biochem. 60: 39-71) but currently lack broad application for other uses. In addition, a separate class of enzymes, termed primase-polymerases (PrimPol) comprise both DNA polymerase and DNA primase activities and may be use both for primer synthesis and replication of a DNA molecule. Said PrimPols, unlike conventional primases, generate DNA primers (Lipps et al., 2003. EMBO J 22: 2516-2525). A PrimPol isolated from *Thermus thermophiles* was recently found to provide excellent results in the amplification of whole-genomes from single cells, when compared to random priming (Picher et al., 2016. Nature Comm 7: 13296).

In the current state of the art, *in vitro* replication of a DNA template, especially large DNA molecules such as whole genomes, is mostly based on multiple displacement amplification (MDA). MDA is an isothermal amplification method employing oligonucleotide primers and a strand-displacing DNA polymerase, such as Phi29 DNA polymerase to exponentially amplify a template DNA molecule ((Dean et al., 2001. Genome Res. 11(6): 1095-1099; Dean et al., 2002. Proc. Natl. Acad. Sci. 99(8): 5261-5266). Phi29 DNA polymerase combines high processivity, strand displacement activity, and 3' to 5' proofreading exonuclease activity resulting in synthesis of DNA fragments >70 kb with very low error rates (Blanco et al., 1989. J Biol Chem 264: 8935-8940; Garmendia et al., 1992. J Biol Chem 267: 2594-2599; Esteban et al., 1993. J Biol Chem 268: 2719-26).

Whereas native DNA commonly exists as a double-helix structure, the use of either specific or degenerate, random oligonucleotides such as hexamer primers requires denatured or single-stranded DNA for efficient priming. Denaturation of DNA by heat or chemical methods can lead to strand breaks, and other harmful damage to the initial template DNA, which is highly undesirable and possibly detrimental to downstream analysis. Moreover, the use of random primers often results in biased amplification of template DNA, in sequencing errors due to mispriming, and/or in non-specific amplification by self-priming of the random oligonucleotides (see for instance Hansen et al., 2010. Nucleic Acids Res 38: e131; van Gurp et al., 2013. PLoS ONE 8(12): e85583; Sabina and Leamon 2015. Methods Mol Biol. 1347:15-41). Some of these problems are in part solved by the use of a PrimPol isolated from *Thermus thermophiles* (Richer et al., 2016. Nature Comm 7: 13296), which possesses intrinsic primase activity without the need for exogenously added oligonucleotides. However, both random oligonucleotides and PrimPol require or favor single stranded or denatured DNA as template for efficient priming. Denaturation can be achieved by elevating the temperature, or by addition of a highly alkaline solution, followed by neutralization of the solution prior to replication. Said additional denaturation steps is prone to artefacts such as damage or breakage of the template DNA, and increase a risk of contamination. Therefore, additional denaturation step is preferably avoided, especially in high throughput DNA replication methods and in cases of delicate or scarce DNA templates. EP2206789 and WO0041524 describe further amplification methods in the absence of a primer.

There is thus a need for a DNA replication method that does not require oligonucleotide primers and/or the additional step of denaturing the DNA template molecule.

### 2 BRIEF DESCRIPTION OF THE INVENTION

Provided herein are methods, and use of compositions and kits for amplification of a template or target DNA in the absence of, or with reduced amounts of, oligonucleotide primers. The target DNA may be a circular molecule such as a plasmid, a cosmid, or a circularized DNA padlock probe, or a linear molecule such as a genome, one or more genomic fragments, or synthetically or enzymatically generated nucleic acid fragments. A template DNA molecule may be in a single-stranded or double-stranded form.

The invention provides a method for amplifying a template DNA molecule, comprising a) providing a template DNA molecule; b) providing a promoter-dependent RNA polymerase of the family of single-subunit RNA polymerases, a strand-displacing DNA-dependent DNA polymerase and a combination of ribonucleotides and deoxyribonucleotides; c) incubating said materials in a suitable buffer and for a suitable amount of time to allow replication and amplification of said template DNA molecule, wherein priming by said promoter-dependent RNA polymerase occurs independently of a consensus promoter sequence.

Said template DNA molecule, RNA polymerase, DNA polymerase, and nucleotides are preferably incubated in said suitable buffer at constant temperature within a range of 3°C., 6°C., or 12°C for a suitable amount of time.

Said RNA polymerase is a member of the family of single subunit RNA polymerases. A preferred RNA polymerase is selected from the group of T7 RNA polymerase, T3 RNA polymerase, SP6 RNA polymerase, or variants thereof.

Said DNA-dependent DNA polymerase has strand-displacement activity. A preferred DNA polymerase is selected from Phi29, Bst, Vent DNA polymerase, or a combination or variants thereof.

In a preferred method of the invention, said ribonucleotides comprise at most three ribonucleotides, of which at least one ribonucleotide has a purine nucleobase, such as adenine or guanine, or a combination thereof.

Replication and amplification of a template DNA molecule by the methods of the invention may further be performed in the presence of at least one oligonucleotide primer complementary to the template DNA molecule, a mix of random oligonucleotide primers, or a combination thereof.

In methods of the invention, one of the nucleotides or ribonucleotides, preferably at least one species of the nucleotides or ribonucleotides, is modified or labeled, preferably with a detectable label.

An amplified product that is replicated and amplified by the methods of the invention, may be directly or indirectly detected by fluorescence and/or by chemical means, for example by a biotin- or fluorophore-labeled probe, and/or by amplification in the presence of a fluorophore-conjugated dNTP.

The invention further provides use of an RNA polymerase of the family of single-subunit RNA polymerases, for initiating priming on a DNA template and, optionally, amplifying the DNA template from the primer by a strand-displacing DNA-dependent DNA polymerase in the presence of deoxyribonucleotides, wherein initiating of priming occurs in the presence of at most three ribonucleotides, of which at least one ribonucleotide with has a purine nucleobase. Said initiating of priming preferably includes providing at least one ribonucleotide with a purine nucleobase, more preferably 2 or 3 ribonucleotides selected from GTP, ATP, and CTP.

Said RNA polymerase according to the invention may be used for amplifying a single- or double stranded template DNA molecule in a linear or circular form, including genomic DNA. Use of an RNA polymerase according to the invention may be employed for therapeutic purposes, including but not limited to gene therapy and vaccines, or for forensic or diagnostic purposes, for example in genotyping, DNA sequencing, detection of viral or bacterial DNA, or detection of a nucleic acid mutation.

Said promoter-dependent RNA polymerase of the family of single-subunit RNA polymerases preferably is selected from the group of T7 RNA polymerase, T3 RNA polymerase, SP6 RNA polymerase, or variants thereof.

The invention further provides a use of a kit of parts, comprising a promoter-dependent RNA polymerase of the family of single-subunit RNA polymerases, a strand-displacing DNA-dependent DNA polymerase, or a mixture thereof, at most 3 ribonucleotides of which at least one ribonucleotide with has a purine nucleobase, deoxyribonucleotides, and optionally, a part holding a suitable buffer, for amplification of a template DNA molecule.

The invention further provides use of a kit of parts according to the invention for amplification of a template DNA molecule, wherein the promoter-dependent RNA polymerase of the family of single-subunit RNA polymerases is selected from the group of T7 RNA polymerase, T3 RNA polymerase, SP6 RNA polymerase, or variants thereof.

### 3 FIGURE LEGENDS

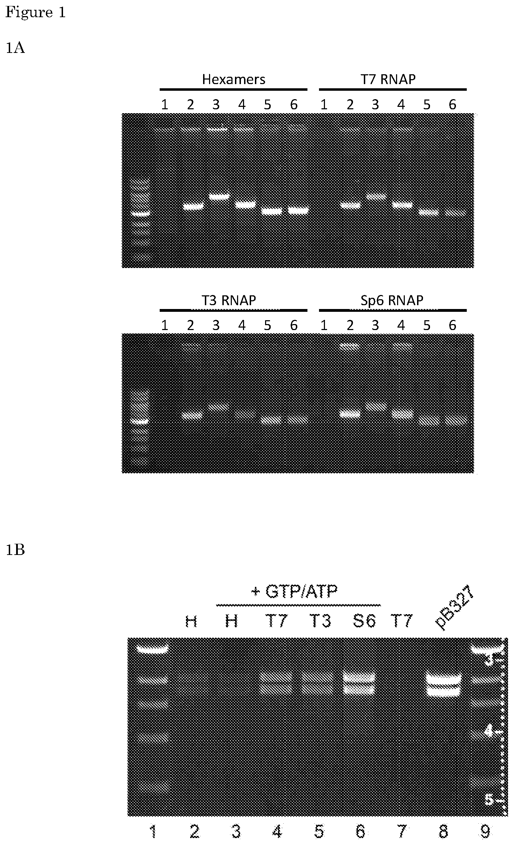

Figure 1. Priming of DNA amplification by RNA polymerases. Fig. 1A. Amplification products of circular plasmid DNA following priming by random hexamers or by different RNA polymerases. Priming by RNA polymerases occurs in the absence of exogenously added oligonucleotide primers and appears independent of their cognate promoter sequences. Templates used: Lanes 1, no template control; Lanes 2, p53 (no RNA polymerase promoter); Lanes 3: plasmid p3 comprising a single T7 promoter; Lanes 4, plasmid pB327 comprising two inverted T7 promoters; Lanes 5, plasmid pBSK comprising T3 and T7 promoters; Lanes 6, pGem-7 plasmid comprising T7 and SP6 promoters. **Fig. 1B****.** Restriction enzyme analysis (BsaI) illustrates faithful amplification of pB327 plasmid DNA following priming by random hexamers or by different RNA polymerases in the presence or absence of purinergic ribonucleotides. Lane 1, marker; Lane 2, hexamer primers (H); Lane 3, hexamer primer and GTP/ATP; Lane 4, T7 RNA polymerase and GTP/ATP; Lane 5, T3 RNA polymerase and GTP/ATP; Lane 6, SP6 RNA polymerase and GTP/ATP; Lane 7, T7 RNA polymerase without GTP/ATP; Lane 8, digestion control, 250ng pB327 template.

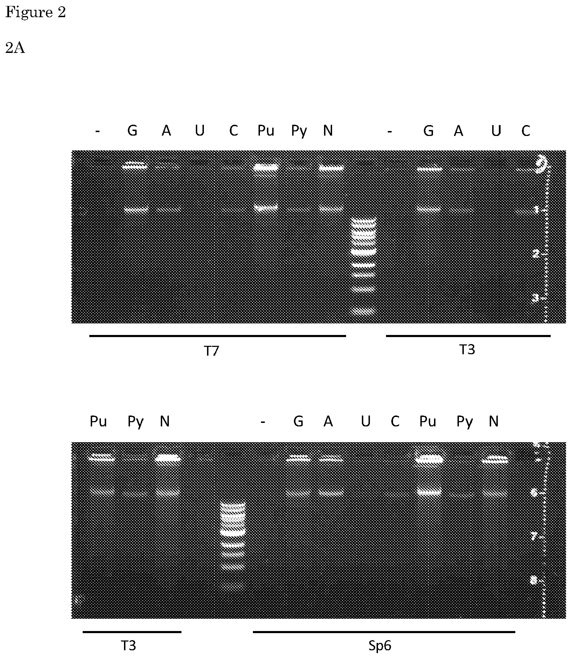

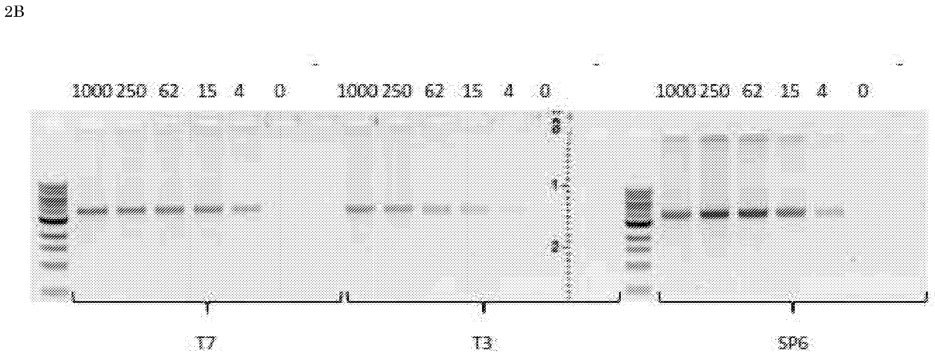

Figure 2. Priming by RNA polymerases in the presence of different ribonucleotides. **Fig. 2A****.** Ribonucleotide dependency of RNA polymerase mediated priming of p53 DNA amplification. Lanes -, no ribonucleotides; Lanes G, 0.5mM GTP; Lanes A, 0.5mM ATP; Lanes U, 0.5mM UTP; Lanes C, 0.5mM CTP; Lanes Pu, 0.25mM GTP and 0.25mM ATP; Lanes Py, 0.25mM UTP and 0.25mM CTP; Lanes N, 0.125mM of all four ribonucleotides. **Fig. 2B****.** DNA amplification of plasmid pB327 in conditions with RNA polymerases and decreasing ribonucleotide concentrations. Concentrations of GTP and ATP in micromolar are indicated above each lane. (-) depicts no template controls containing 1 mM GTP and ATP.

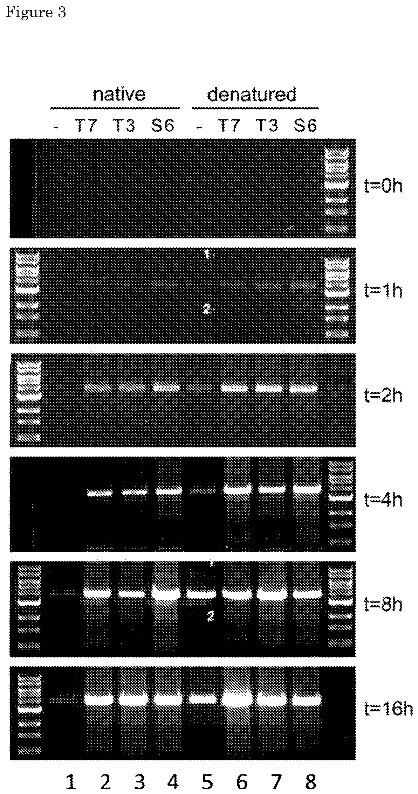

Figure 3. Amplification of native or denatured pB327 plasmid DNA in the presence of RNA polymerases at different incubation times. Reactions in lanes 1-4 contained 10 ng pB327 native, non-denatured plasmid DNA as starting template, Lanes 5-8 contained 10 ng pB327, denatured by heat prior to amplification. All reactions comprise 50 µM hexamers, in addition to the RNA polymerase indicated above each lane.

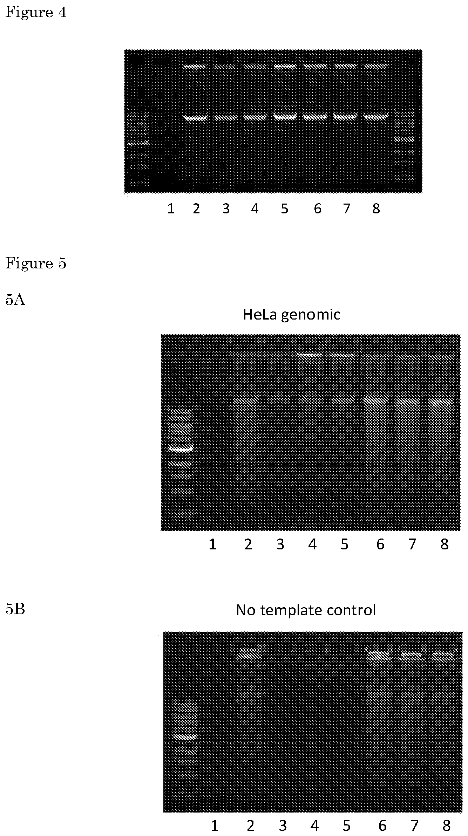

Figure 4. Amplification of single-stranded, circular M13mp18 DNA template followed by XbaI digestion. Lower band represents correct product migrating around 7 kb. Lane 1, no-priming control; Lane 2, 50µM hexamer primers; Lane 3, 0.5 U/µl T7 RNA polymerase; Lane 4, 0.5 U/µl T3 RNA polymerase; Lane 5, 0.5 U/µl SP6 RNA polymerase; Lane 6, 0.5 U/µl T7 RNA polymerase and 50µM hexamer primers; Lane 7, 0.5 U/µl T3 RNA polymerase and 50µM hexamer primers; Lane 8, 0.5 U/µl SP6 RNA polymerase and 50µM hexamer primers.
Figure 5. Specific amplification of genomic DNA. **Fig. 5A****.** Amplification of 1ng/µl HeLa genomic DNA template. **Fig. 5B****.** Non-specific amplification by hexamer primers in the absence of template. Lanes 1, no priming control; Lanes 2, 50 µM hexamer primers; Lanes 3, 0.5 U/µl T7 RNA polymerase; Lanes 4, 0.5 U/µl T3 RNA polymerase; Lanes 5, 0.5 U/µl SP6 RNA polymerase; Lanes 6, 0.5 U/µl T7 RNA polymerase and 50µM hexamer primers; Lanes 7, 0.5 U/µl T3 RNA polymerase and 50µM hexamer primers; Lanes 8, 0.5 U/µl SP6 RNA polymerase and 50µM hexamer primers.

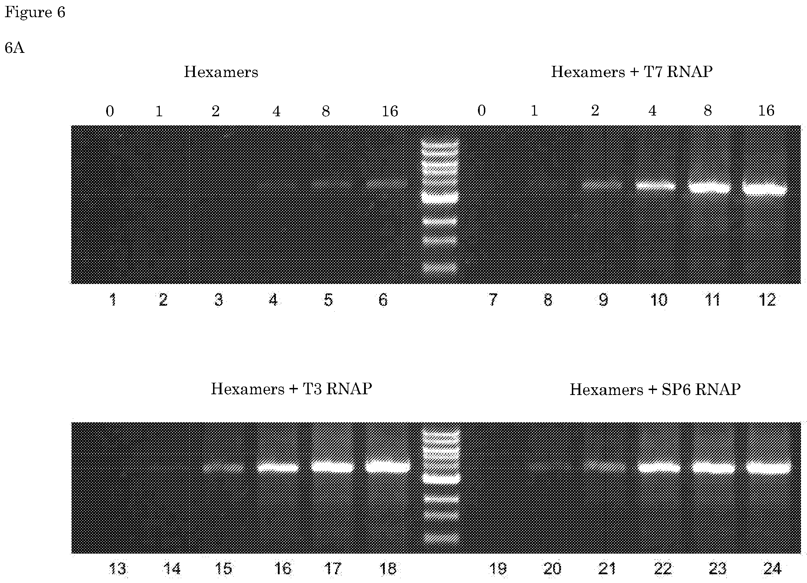

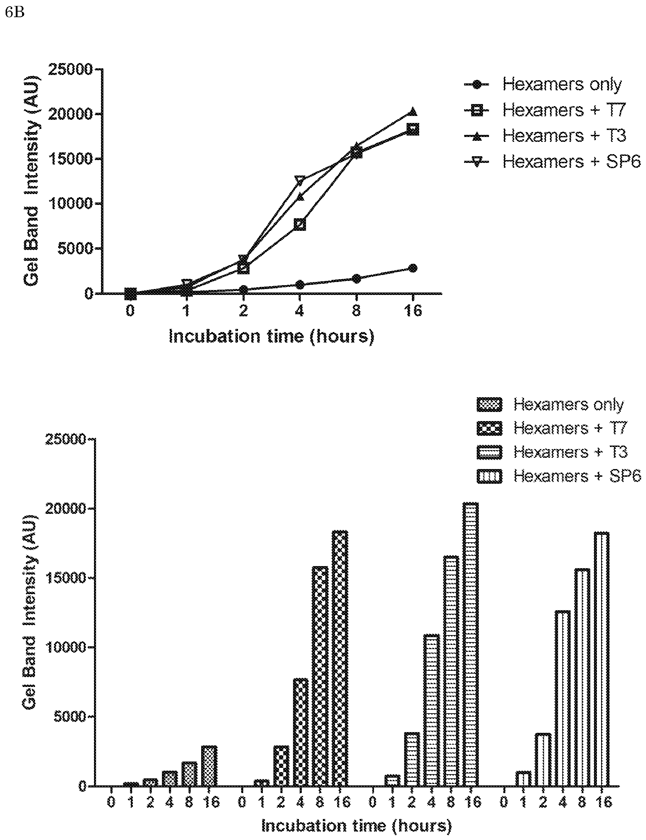

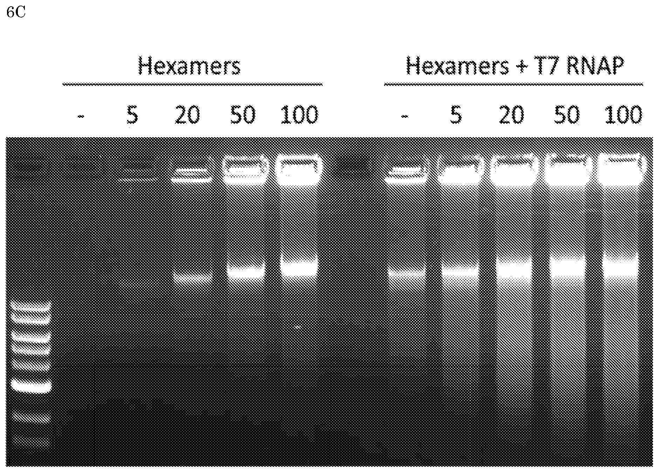

Figure 6. RNA polymerases enhance and increase yield of hexamer-primed amplification of circular DNA templates. **Fig. 6A****.** Enhanced amplification of native p53 plasmid DNA in the presence of RNA polymerases (RNAP). Indicated above the lanes is the amplification time in hours. **Fig. 6B****.** (top and bottom) Quantification of DNA amplification in time and the relative activities of RNA polymerases. **Fig. 6C****.** Enhanced amplification of denatured p53 plasmid DNA mediated by RNA polymerases in the presence of the indicated hexamer concentrations (in micromolar). Template used was 0.5ng/pl denatured p53 plasmid DNA, which does not comprise a T7, T3, or SP6 promoter sequence.

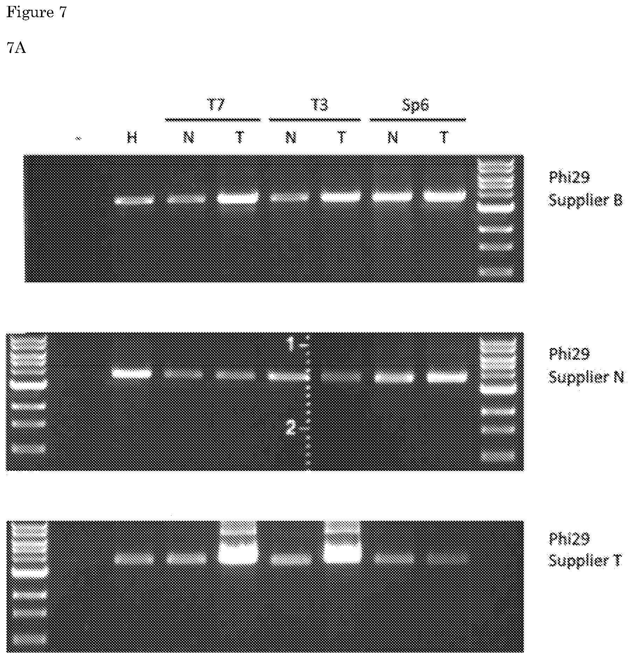

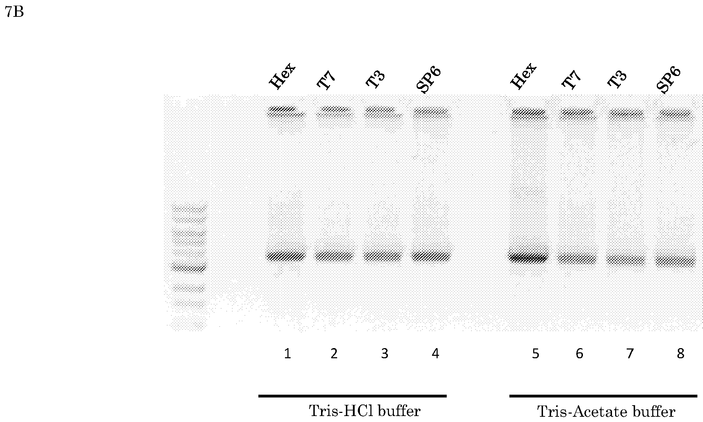

Figure 7. DNA amplification mediated by RNA polymerases and Phi29 obtained from different commercial suppliers. **Fig. 7A****.** RNA polymerases and phi29 DNA polymerase from different suppliers. Upper panel, Phi29 from Biolab Innovative Research Technologies (supplier B); middle panel, Phi29 from New England Biolabs (supplier N); lower panel, Phi29 from Thermo Fisher (supplier T). Lanes - are no priming controls, Lanes H denote hexamer priming. T7, T3, and SP6 RNA polymerases obtained from New England Biolabs (N) or from Thermo Fisher (T) were used as indicated above each lane. Template used in each reaction was 0.5 ng/µl p53 plasmid DNA, which does not comprise a T7, T3, or SP6 promoter sequence. **Fig. 7B****.** Amplification in the presence of different buffer systems.

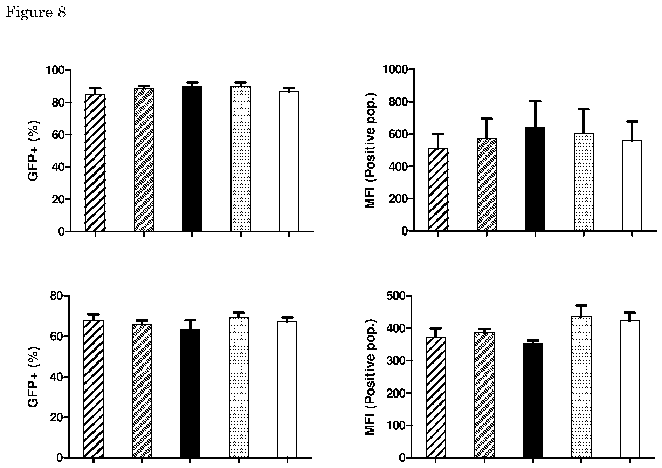

Figure 8. Transfection efficiency and expression levels of DNA amplified under different conditions as indicated. Upper panels illustrate transfection efficiency (fraction of GFP-expressing cells in the total cell population) and mean fluorescent intensity (MFI) for human HEK293 cells, lower panel show results for mouse B16F10 cells. Priming of DNA amplification was initiated by random hexamers in the absence (striped white bars) or presence of ribonucleotides (striped grey bars), or by T7 RNA polymerase (black bars), T3 RNA polymerase (grey bars), or SP6 RNA polymerase (white bars) in the presence of ribonucleotides.

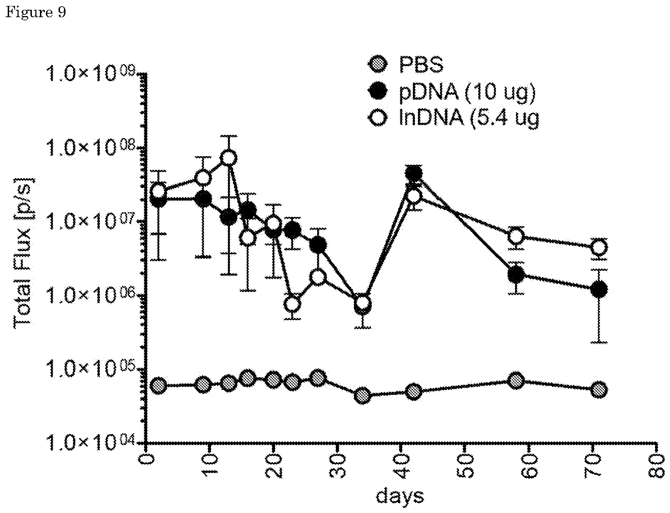

Figure 9. Luciferase activity over time in mice injected with plasmid DNA or linear DNA, versus control mice. The average and standard deviation of 4 mice per group is indicated.

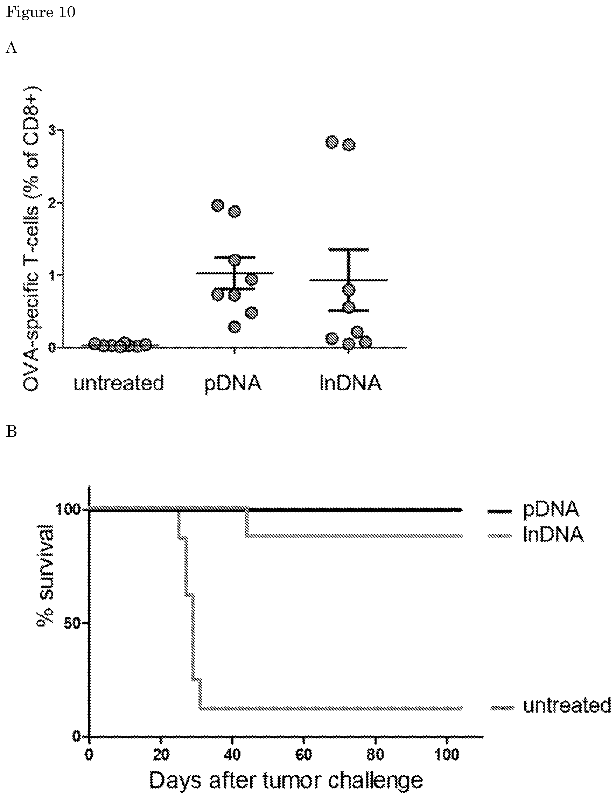

Figure 10. Vaccination with either plasmid DNA or linear DNA. **Fig. 10A****.** OVA epitope-specific T cells as a percentage of the total number or CD8-positive T cells, as induced by plasmid DNA or linear DNA, versus control. **Fig. 10B****. The** Kaplan-Meier plots of vaccinated mice that were challenged with B16-OVA tumor cells at 21 days post-vaccination.

### 4 DETAILED DESCRIPTION OF THE INVENTION

### 4.1 Definitions

The term "template DNA molecule", " or "DNA template", as is used herein, refers to a DNA molecule that is to be replicated. Said DNA template can be any DNA molecule, including a single or double stranded DNA molecule, a linear or circular DNA molecule, and a small or a large DNA molecule, ranging from linear or circular plasmid molecules of less than 10 kilo bases to large DNA molecules such genomic DNA molecules or BAC plasmids.

The term "suitable buffer", as is used herein, refers to an aqueous buffered solution of which the pH is at a nearly constant value. A suitable buffer for replication reactions comprises a divalent metal salts, preferably a magnesium salt, such as magnesium chloride, magnesium sulphate and/or magnesium acetate.

The term "RNA polymerase ", as is used herein, refers to an DNA-dependent RNA polymerase enzyme that produces a DNA templated RNA transcript. Said RNA polymerase is a member of the family of single-subunit RNA polymerases that includes many phage RNA polymerases (T7, T3, K11, SP6, N4, and others) as well as the mitochondrial RNA polymerases (McAllister and Raskin, 1993. Mol Microbiol. 10: 1-6). A preferred RNA polymerase is selected from the group of T7 RNA polymerase, T3 RNA polymerase, SP6 RNA polymerase, or variants thereof. Such variants include for instance mutant T7 RNA polymerases capable of utilizing both canonical and non-canonical ribonucleotides and deoxynucleotides as substrates (Kostyuk et al., 1995. FEBS Lett. 369: 165-168; Sousa et al., 1995. EMBO J. 14(18): 4609-4621; Gudima et al., 1998. FEBS Lett. 439: 302-306; Padilla et al., 2002. Nucl. Acids Res. 30(24): e138), RNA polymerase variants displaying higher thermostability (Hi-T7™ RNA Polymerase from New England Biolabs; Boulain et al., 2013. Protein Eng Des Sel. 26(11): 725-734), or a mutant RNA polymerase with decreased promoter specificity (Ikeda et al., 1993. Biochemistry 32(35):9115-9124).

The term "DNA polymerase ", as is used herein, refers to an DNA dependent DNA polymerase enzyme that produces a DNA templated DNA molecule. Said DNA polymerase adds deoxyribonucleotides to the 3'-end of a DNA strand.

The term "strand-displacing DNA polymerase ", as is used herein, refers to an DNA polymerase which is able to displace a downstream DNA strand. Hence, said enzyme is able to unwind a double stranded DNA molecule during replication of said molecule. Said polymerase preferably lacks a 5'→3' exonuclease activity. A suitable strand-displacing DNA-dependent DNA polymerase includes the Klenow fragment of DNA polymerase I, a Klenow fragment of a *Bacillus stearothermophilus* DNA polymerase termed Bst polymerase (New England Biolabs, Ipswich, MA), Bsm DNA Polymerase, large fragment (Thermo Fisher Scientific, Waltham, MA), BcaBEST DNA polymerase (Takara Bio, Kusatsu, Japan), *Thermoanaerobacter thermohydrosulfuricus* (Tts) DNA polymerase ( US 5,744,312 ), a DNA polymerases from *Thermus aquaticus, Thermus flavus* or *Thermus thermophiles,* a variant of T7 DNA-dependent DNA polymerase termed SEQUENASE (Thermo Fisher Scientific, Waltham, MA), a T5 DNA dependent DNA polymerase (Fujimura and Roop, 1976. J Biol Chem 251: 2168-2174), aT4 DNA polymerase holoenzyme (Kaboord and Benkovic, 1995. Curr Biol 5:149-157), a DNA dependent DNA polymerase from *Thermococcus litoralis,* termed VENT polymerase (New England Biolabs, Ipswich, MA), phage M2 DNA polymerase (Matsumoto et al., 1989. Gene 84: 247), phage PRD1 DNA polymerase (Jung et al., 1987. Proc Natl Aced Sci USA 84: 8287; Zhu and Ito, 1994. Biochim Biophys Acta 1219: 267-276), Phi29 DNA polymerase, and any strand-displacing DNA-dependent DNA polymerase mutant thereof including, for example, a fusion of a polymerase with a DNA binding protein (de Vega et al., 2010. PNAS 107: 16506-16511).

The term "ribonucleotide", as is used herein, refers a molecule comprising a ribose sugar group, a nucleobase and at least one phosphate group. Said nucleobase includes adenine, guanine, cytosine, uracil, and any modifications thereof. The nucleobases adenine and guanine are collectively termed purines, while cytosine and uracil are collectively termed pyrimidines. The term ribonucleotide includes reference to an analogue of a ribonucleotide such as a fluorescent molecule, for example 1,3-diaza-2-oxophenothiazine-ribose-5'-triphosphate (tCTP), and/or other analogues such as inosine, xanthosine, N4-hydroxycytosine, N4-methoxycytosine and 6H, 8H-3,4-dihydropyrimido[4,5-c][1,2]oxazin-7-one (Suzuki et al., 2005. Nucleic Acids Symp Series 49: 97-98).

The term "deoxyribonucleotide", as is used herein, refers a molecule comprising a deoxyribose sugar group, a nucleobase and at least one phosphate group. Said nucleobase includes the naturally occurring adenine, guanine, cytosine, thymidine, and any modifications thereof. The nucleobases adenine and guanine are collectively termed purines, while cytosine and thymidine are collectively termed pyrimidines. The term deoxyribonucleotide includes reference to an analogue of a deoxyribonucleotide such as a deoxyuridine, deoxyinosine, and/or deoxyxanthosine.

The term "nucleotide", as is used herein, refers to a ribonucleotide, a deoxyribonucleotide, or any variant or modification thereof. It will be appreciated that a great variety of nucleotide modifications have been made and described that serve many useful purposes known to those skilled in the art. The term nucleotide as it is employed herein embraces such chemically, enzymatically or metabolically modified forms of nucleotides.

The term "primer", as is used herein, refers to an oligonucleotide that is effective in annealing to a template DNA and priming replication of said template DNA by a DNA polymerase. Said oligonucleotide may be added exogenously to a template DNA and may comprise deoxyribonucleotides, ribonucleotides, or a combination or variants thereof. Alternatively, a primer may be generated from a DNA template by an RNA polymerase. Said primer can be generated by an RNA polymerase or variant thereof in the presence of one or more nucleotides. Said nucleotides preferably comprise ribonucleotides, preferably 1 to 3 species selected from GTP, ATP, and CTP, more preferably comprising at least one purine nucleobase, such as adenine or guanine, or both adenine and guanine nucleobases. Said variants include synthetic oligonucleotide analogues such as phosphorothioate, phosphotriester, phosphorothioate 2-alkylated, and phosphoramidate analogues, analogues with modifications at the 2'-position of nucleoside sugar rings such as 2'-fluoro, O-methyl, or methoxyethyl, peptide nucleic acid, bridged nucleic acid, and/or locked nucleic acid molecules.

The term "padlock probe", as is used herein, refers to an oligonucleotide of which the ends are complementary to adjacent sequences on a target template RNA or DNA molecule. Hybridization of a padlock probe to its target allows circularization by ligation. A circularized padlock probe may subsequently serve as template DNA for amplification.

### 4.2 Methods of the invention

The invention is based on the unexpected observation that a RNA polymerase of the family of single-subunit RNA polymerases such as T7 RNA polymerase is able to efficiently mediate priming of both native and denatured DNA templates for subsequent amplification by a DNA-dependent DNA polymerase, in the absence of a consensus T7 promoter sequence and exogenously added oligonucleotide primers. It was subsequently found that also other RNA polymerases such as SP6 and T3 RNA polymerase are able to initiate DNA amplification in the absence of a consensus SP6 or T3 promoter sequence, respectively. Additionally, said RNA polymerases are shown to initiate priming on single stranded DNA templates. The unexpected nature of the findings lies in the fact that transcriptional activity of DNA dependent RNA polymerases like T7, T3, and SP6 RNA polymerases has been widely described as being highly specific for their respective promoter sequences, and that binding of said RNA polymerases only occurs on double stranded DNA promoter sequences (Golomb et al., 1977. J Virol 21: 743-752; Stump and Hall, 1993. Nucleic Acids Res 21: 5480-5484; Maslak and Martin, 1993. Biochemistry 32: 4281-4285; Li et al., 1996. Biochemistry 35: 3722-3727).

Priming by said RNA polymerase according to methods of the invention is dependent on the presence of at least one ribonucleotide. In order to prevent prolonged polymerization of RNA transcripts, the reaction preferably is performed in the presence of at most 3 ribonucleotides. Said at most 3 ribonucleotides preferably include at least one purine ribonucleotide, such as ATP or GTP, or a combination thereof. It is preferred that either CTP and/or UTP is left out of the reaction mixture.

The invention therefore provides a method for amplifying a template DNA molecule, comprising a) providing a template DNA molecule; b) providing a promoter-dependent RNA polymerase of the family of single-subunit RNA polymerases, a strand-displacing DNA-dependent DNA polymerase and a combination of ribonucleotides and deoxyribonucleotides; and c) incubating said materials in a suitable buffer and for a suitable amount of time to allow replication and amplification of said template DNA molecule, and, optionally, d) detecting the amplified product.

Said promoter-dependent RNA polymerase of the family of single-subunit RNA polymerases mediates priming for a subsequent elongation reaction by a DNA polymerase. Without being bound by theory, said RNA polymerase binds to template DNA and binds a single RNA nucleotide that may serve as a primer for elongation by a DNA-dependent DNA polymerase. Alternatively, or in addition, said RNA polymerase produces a short stretch of RNA by a process termed abortive transcription, in which the RNA polymerase binds to a DNA molecule and starts synthesis of short mRNA transcripts. Said abortive transcription may involve DNA scrunching (Revyakin et al., 2006. Science 314: 1139-1143). The short, abortive mRNA transcripts can be used as a ribonucleotide primer and are elongated by a DNA-dependent DNA polymerase.

A preferred RNA polymerase is a T3 RNA polymerase, SP6 RNA polymerase and a T7 RNA polymerase. A most preferred RNA polymerase is a T7 RNA polymerase.

Said strand-displacing DNA-dependent DNA polymerase preferably is Phi29, Bst, and/or Vent DNA polymerase. The optimal temperature of Phi29 polymerase is about 30 °C. The optimal temperature of Bst polymerase is 60-65°C, while the optimal temperature of Vent polymerase is about 72 °C. As most natural RNA polymerase are active at 30-37 °C, the enzyme of choice for combining with a T3 RNA polymerase, SP6 RNA polymerase or a T7 RNA polymerase is Phi29 polymerase. However, it will be clear to a person of skill in the art that a more thermostable DNA-dependent RNA polymerase may be combined with a Bst polymerase or a Vent polymerase at a higher incubation temperature.

Phi29 DNA polymerase has a higher processivity and strand displacing ability, when compared to the Bst and/or Vent DNA polymerase. In addition, Phi29 DNA polymerase possesses proof-reading activity, resulting in highly accurate replication (Garmendia et al., 1992. J. Biol. Chem. 267, 2594-2599). A most preferred strand-displacing DNA-dependent DNA polymerase, therefore, is Phi29 polymerases. However, other strand-displacing DNA-dependent DNA polymerase that are or will be known may also be suited for use in the methods of this invention.

A method of the invention is performed for a suitable amount of time to amplify the DNA template. Said amount of time preferably is 0.5-48 hours, more preferably 1-24 hours, more preferred 2-20 hours, such as at least 5 hours, at least 8 hours, at least 12 hours, or at least 16 hours.

A method of the invention is performed in a suitable buffer. Said buffer preferably comprises a buffering agent to keep the pH at a nearly constant value. Said buffering agent may include phosphate, borate, N-cyclohexyl-2-aminoethanesulfonic acid (CHES), tris(hydroxymethyl)aminomethane (Tris), 2-(N-morpholino)ethanesulfonic acid (MES), glycine, and/or [tris(hydroxymethyl)methylamino]propanesulfonic acid (TAPS). A preferred reaction pH for a polymerase is between 6 and 10, preferably between 7 and 9, such as 7.2, 7.5, 7.8, 8.0, 8.1, 8.5. 8.6 or 8.8.

A preferred buffering agent is or comprises Tris, preferably 10-100 mM Tris. Said Tris preferably is set at a desired pH by the addition of an acid such as acetate and/or hydrogen chloride.

A suitable buffer for replication reactions further comprises divalent metal ions such as a Mg2+ or Mn2+. Divalent metal ions may be provided as salts thereof such as magnesium chloride, magnesium sulphate and/or magnesium acetate. Said concentration preferably is between 0.5 and 20 mM, such as between 1 and 10 mM, preferably about 5-10 mM.

Additional components of said buffer may include potassium ions, such as potassium chloride, other salts such as ammonium sulphate, and/or betaine, ethylene glycol, 1,2-propanediol and/or spermidine, as known in the art to enhance replication of certain DNA templates, such as 2-10% DMSO or 2-10% glycerol (Cheng et al., 1994. Proc Natl Acad Sci 91: 5695-5699).

Preferably, additional ingredients are included in the reaction buffer to stabilize enzyme activity including gelatin, albumin, reducing agents such as betamercaptoethanol, dithiothreitol (DTT), and/or tris(2-carboxyethyl)phosphine (TCEP), and a mild detergent such as, for example, TWEEN 20 or Triton-X 100..

In an embodiment, a preferred buffer comprises Tris-acetate, magnesium acetate, and potassium acetate, more preferred 33 mM Tris-acetate, pH 7.9 at 37 °C, 10 mM magnesium acetate, 66 mM potassium acetate, 0.1% (w/w) Tween 20, and 1 mM DTT.

In an embodiment, a preferred buffer comprises Tris-chloride, magnesium chloride and ammonium sulphate, more preferred 50 mM Tris-HCl (pH 7.5 at 25°C), 10 mM MgCl₂, 10 mM (NH4)₂SO₄, and 4 mM DTT.

A method of the invention may further include the provision of at least one exogenous oligonucleotide as a primer, which oligonucleotide is complementary to a stretch of nucleotides on the template DNA molecule, a mixture of random oligonucleotides, or a combination thereof. Said oligonucleotide or mixture of oligonucleotides preferably is or comprises one or more single stranded nucleic acid molecules, preferably DNA molecules, RNA molecules, or mixtures or analogues thereof. Said single stranded nucleic acid molecules preferably comprise 6-100 bases in length, such as 9-30 bases. Said single stranded nucleic acid molecules may serve as additional starting points for replication and amplification of the template DNA molecule.

An amplified product that is produced by the methods of the invention may be detected and visualized, for example by gel electrophoresis. Gel electrophoresis is a technique used to separate DNA molecules based on their size, as all DNA molecules essentially have the same charge. Electrophoresis involves running a current through a gel containing the molecules of interest. Based on their size, the molecules will travel through the gel at different speeds, allowing them to be separated from one another.

Said gel typically is an agarose or polyacrylamide gel. Polyacrylamide gels are usually used for small DNA fragments of DNA of up to 500-1000 base pairs (bp). Agarose gels can be used for DNA fragments of 100-20 kbp, but resolution of over 6 Mb can be achieved with pulsed field gel electrophoresis (PFGE).

As an alternative, or in addition, an amplified product that is produced by the methods of the invention may be detected and visualized by DNA-binding or DNA-intercalating agents or dyes such as ethidium bromide, crystal violet, Hoechst stains or DAPI (4',6-diamidino-2-phenylindole), cyanine dyes such as SYBR Green, or Eva Green. Alternatively, an amplified product that is produced by the methods of the invention may be detected by using a conjugated or fluorescent probe, such as for example by a biotin- or fluorophore-tethered complementary oligonucleotide or fluorescently-labeled molecular beacon, and/or by amplification in the presence of a modified or conjugated dNTP.

Examples of suitable fluorophores include, but are not limited to, Atto425 (ATTO-TEC GmbH, Siegen, Germany), Atto 647N (ATTO-TEC GmbH, Siegen, Germany), YakimaYellow (Epoch Biosciences Inc, Bothell, WA, USA), Cal610 (BioSearch Technologies, Petaluma, CA, USA), Cal635 (BioSearch Technologies, Petaluma, CA, USA), FAM (Thermo Fisher Scientific Inc., Waltham, MA USA), TET (Thermo Fisher Scientific Inc., Waltham, MA USA), HEX ((Thermo Fisher Scientific Inc., Waltham, MA USA), cyanine dyes such as Cy5, Cy5.5, Cy3, Cy3.5, Cy7 (Thermo Fisher Scientific Inc., Waltham, MA USA), Alexa dyes (Thermo Fisher Scientific Inc., Waltham, MA USA), Tamra (Thermo Fisher Scientific Inc., Waltham, MA USA), ROX (Thermo Fisher Scientific Inc., Waltham, MA USA), JOE (Thermo Fisher Scientific Inc., Waltham, MA USA), fluorescein isothiocyanate (FITC, Thermo Fisher Scientific Inc., Waltham, MA USA), and tetramethylrhodamine (TRITC, Thermo Fisher Scientific Inc., Waltham, MA USA).

As is known to a person skilled in the art, said fluorophore can be detected using any suitable method known in the art. For example, a fluorophore can be detected by exciting the fluorophore with the appropriate wavelength of light and detecting the emitted fluorescence.

A template DNA molecule for amplification according to the methods of the invention may be any linear or circular DNA molecule, including mitochondrial DNA and genomic DNA from bacteriophages, viruses, bacteria, including archaebacterial, protozoa such as Amoebozoa, Choanozoa, and Excavata, Chromista, including algae, and diatoms, plants, fungi, animals, including human. Amplification of said genomic template, preferably whole genomic template, may be used for biomedical and forensic applications. Genomic analyses, such as comparative genomic hybridization, genotyping of polymorphic loci and detection of disease gene mutations, are important in genomic medicine and forensic science.

Many biomedical and forensic DNA analyses techniques require nanogram to microgram quantities of genomic DNA. DNA samples often have to be amplified before genomic analyses can be performed. The methods of the invention may be used for such whole genome amplification of a genomic template DNA molecule.

As an alternative, or in addition, the methods of the invention may be employed to amplify a circular template DNA molecule. Said circular DNA template is, for example, a recombinant DNA plasmid, a naturally occurring plasmid, a mitochondrial genome, or a circular genome of some bacteriophages and viruses. Said circular DNA template may also be artificially generated, for example by use of padlock probes, by gene synthesis, or by recombinant DNA methods including enzymatic ligation e.g. by T4 DNA ligase, or template-free ligation using special DNA ligases such as a ligase that is capable of template-independent, intramolecular ligation of a single-stranded DNA sequence to generate the single-stranded DNA circle e.g. CircLigase (Lucigen Corp., Middleton, WI). As will be appreciated by those skilled in the art, a linear DNA template product can also be converted into a circular template using for example a DNA recombinase, a protelomerase, a resolvase or integrase, by self-ligation or by ligation of terminal hairpin loops.

Amplification methods such as the amplification methods of this invention, are ideally suited for generating DNA templates for RNA synthesis or *in vitro* transcription, including linear templates. For instance, a circular DNA construct comprising a promoter for an RNA polymerase, for example an SP6, T3 or T7 promoter, and optionally a poly(A) sequence, may be amplified in the presence of a DNA polymerase and an RNA polymerase (either matching the promoter site or not) according to the present invention. A restriction enzyme may be present during the amplification reaction that digests the amplified products, resulting in discrete, linearized amplified products suitable for *in vitro* or *in vivo* transcription. Said restriction enzyme preferably is selective for the amplified products, but does not restrict the template DNA molecule. Such selectivity is, for example, provided by a methylation-sensitive restriction enzyme. For example, a template DNA molecule that is produced in a Dam+ (methylates A in recognition sequence GATC) *E. coli* strain, can not be restricted by MboI, while the amplified products are not methylated and can be restricted by this enzyme. As is well known in the art, methylation sites overlapping the recognition site of certain endonuclease (e.g. XbaI, ClaI) may also be used, see international.neb.com/tools-and-resources/usage-guidelines/dam-and-dcm-methylases-of-e-coli, or the restriction enzyme database REBASE (at rebase.neb.com/rebase/rebms.html) for more examples. Similarly, a template DNA molecule that is produced in a Dcm+ (methylates C in CCAGG and CCTGG) *E*. *coli* strain, can not be restricted at the methylated sites by certain restriction enzymes such as StyD4I, ApaI, or FseI, while the amplified products are not methylated and can be restricted by these enzymes.

In addition, amplification methods such as the amplification methods of this invention, are ideally suited for generating linear viral templates. For example, a circular construct comprising a DNA copy of a viral genome may be amplified in the presence of a DNA polymerase and an RNA polymerase, for example a T7 polymerase, nucleotides and ribonucleotides. Said circular construct comprises a restriction enzyme recognition sequence in between the terminal repeat sequences, preferably a Dam, Dcm, or EcoKI methylase-sensitive restriction enzyme recognition sequence. The presence of said restriction enzyme during the amplification reaction will restrict the amplified products, resulting in linearized amplified genomic DNA copies of a viral genome with terminal repeat sequences present at both ends.

### 4.3 Use of the invention

As is indicated herein above, a promoter-dependent RNA polymerase of the family of single-subunit RNA polymerases, such as T7 RNA polymerase, is able to mediate priming on a template DNA for a subsequent elongation reaction by a strand-displacing DNA-dependent DNA polymerase. Surprisingly, this phenomenon occurred even if the DNA template did not comprise a consensus T7 promoter sequence. Also other DNA-dependent RNA polymerases such as SP6 and T3 RNA polymerase were found to initiate replication of a DNA template in the absence of a consensus SP6 or T3 promoter sequence, respectively.

Therefore, the invention provides use of a promoter-dependent RNA polymerase of the family of single-subunit RNA polymerases for providing a primer on a DNA template. Said use according to the invention preferably further comprises amplifying said DNA template from the primer by a strand-displacing DNA-dependent DNA polymerase in the presence of deoxyribonucleotides, preferably in the presence of all four deoxyribonucleotides.

As is indicated herein above, said strand-displacing DNA-dependent DNA polymerase preferably is Phi29, Bst, and/or Vent DNA polymerase.

Said DNA template may be any linear or circular DNA molecule, including plasmid DNA, synthetic DNA, mitochondrial DNA and genomic DNA from bacteriophages, viruses, prokaryotes and eukaryotes, including human.

Amplification of said DNA template may be used for biomedical, diagnostic, forensic, or therapeutic purposes or applications. Genomic analyses, such as comparative genomic hybridization, genotyping of polymorphic loci, detection of disease gene mutations, and DNA sequencing are important in biomedical research, genomic medicine, diagnostics and forensic science. Diagnostic use also includes amplification of DNA templates from viruses, bacteria, and other microbiological agents according to methods of the invention prior to DNA sequencing or detection of amplification products by suitable methods.

Alternatively, or in addition, said use of an RNA polymerase for providing a primer on a DNA template according to the invention is in DNA sequencing, including any method, technology, or process to determine the precise ordering of nucleotides in said template DNA.

Therapeutic applications of DNA, such as gene therapy or DNA vaccines, rely on DNA-based therapeutics including expression vectors, oligonucleotides for antisense or exon-skipping applications, DNA decoys, DNA aptamers, and DNAzymes. Clinical use of said DNA-based therapeutics requires quality controlled, highly pure DNA preparations. Whereas relatively short oligonucleotides may be synthesized chemically to high purity, longer DNA molecules are typically isolated as plasmids from bacterial fermentation cultures. Said plasmids must be isolated through a plurality of different processing steps, including extensive purification steps to eliminate any traces of bacterial genomic DNA, RNA, proteins, and endotoxins. Synthetic, cell-free DNA amplification according to methods of the present invention offer a simple, scalable and affordable alternative for the production of DNA-based therapeutics. In addition, the methods of the invention could easily be adopted to allow incorporation of modified nucleotides in DNA-based therapeutics that may enhance their therapeutic effects.

Alternatively, said use according to the invention is in liquid-phase immunoassays and/or immunohistochemistry. Priming by a DNA dependent RNA polymerase and the subsequent amplification by a strand displacing DNA dependent RNA polymerase can be performed in free solution and on top of immobilized targets (solid phase amplification). Immunohistochemistry is a method that can provide diagnostic and prognostic information to morphological observations and soluble assays. Signal amplification, employing conjugation of a DNA template to a component in a hybridization reaction, followed by priming by a DNA dependent RNA polymerase and the subsequent amplification by a strand displacing DNA dependent RNA polymerase, may be applied to increase sensitivity and specificity of the hybridization reaction.

Attachment of a target template to a solid support may be advantageous and can be achieved through means of a polymer that serves to attach said target template to a solid support. Such solid-state substrates useful in the methods described can include any solid material to which nucleotides can be coupled. This includes materials such as acrylamide, cellulose, nitrocellulose, glass, polystyrene, polyethylene vinyl acetate, polypropylene, polymethacrylate, polyethylene, polyethylene oxide, glass, polysilicates, polycarbonates, teflon, fluorocarbons, nylon, silicon rubber, polyanhydrides, polyglycolic acid, polylactic acid, polyorthoesters, polypropylfumerate, collagen, glycosaminoglycans, and polyamino acids. Solid-state substrates can have any useful form including thin films or membranes, beads, bottles, dishes, fibers, woven fibers, shaped polymers, particles and microparticles. A preferred form for a solid-state substrate is a glass slide or a microtiter plate, such as a standard 96-well plate. Preferred embodiments utilize glass or plastic as a support.

Said DNA template preferably is a circular DNA template, allowing rolling circle amplification to enhance sensitivity and specificity of immunoassays and/or in immunohistochemistry. The resulting high signal-to-noise ratio makes it suitable for detecting, quantifying and visualizing low abundance proteinaceous markers, viral and bacterial DNA in clinical samples, and as an on-chip signal amplification method for nucleic acid hybridization, for example in DNA and RNA microarray assays.

In addition, the amplification techniques as presented herein can be applied to the construction of DNA nanostructures and DNA hydrogels.

### 4.4 Kit of parts

The invention further provides a use of a kit of parts, comprising a part holding a promoter-dependent RNA polymerase of the family of single-subunit RNA polymerases, a part holding a strand-displacing DNA-dependent DNA polymerase, or a combination of said enzymes into a single ready-to-use solution, at most 3 ribonucleotides of which at least one ribonucleotide with has a purine nucleobase, deoxyribonucleotides, and optionally, a part holding a suitable buffer, for amplification of a template DNA molecule. Said ribonucleotides preferably comprises ATP, GTP, or both ATP and GTP.

Said kit optionally further includes the provision of a primer comprising at least one oligonucleotide that is complementary to a stretch of nucleotides on a template DNA molecule, a mixture of random oligonucleotides, or a combination thereof.

Said kit preferably further comprises instructions for use of the promoter-dependent RNA polymerase of the family of single-subunit RNA polymerases and the strand-displacing DNA-dependent DNA polymerase for priming and subsequent amplification of a DNA template.
. Said DNA template may be any linear or circular DNA molecule, including plasmid DNA, mitochondrial DNA and genomic DNA from bacteriophages, viruses, bacteria, including archaebacterial, protozoa such as Amoebozoa, Choanozoa, and Excavata, Chromista, including algae, and diatoms, plants, fungi and animals, including human. Amplification of said genomic template, preferably whole genomic template, may be used for biomedical and forensic applications, including diagnostic applications.

The present invention is further described by reference to the following examples, which are presented herein for illustration only and should not be construed as limiting the invention in any way.

### 5. EXAMPLES

### Example 1

### General Materials and Methods.

*Reagent suppliers*: New England Biolabs (NEB; Ipswich, MA, USA) for T7 RNA polymerase (M0251), T3 RNA polymerase (M0378), SP6 RNA polymerase (M0207), Phi 29 DNA polymerase (M0269), ribonucleotides (N0450), dNTP mix (N0447), XbaI (R0145, 20 U/µl), BsaI-HFv2 (R3733, 20 U/µl), PvuI-HF (R3150, 20 U/µl); Biolab Innovative Research Technologies (BLIRT; Gdańsk, Poland) for Phi29 DNA polymerase (EN-20); Thermo Fisher Scientific (TF, Bleiswijk, the Netherlands) for T7 RNA polymerase (EP0111), T3 RNA polymerase (EP0101), SP6 RNA polymerase (EP0131), and Phi 29 DNA polymerase (EP0091); Commercially available stock buffers supplied with the enzymes and used in some of the Examples are shown below. Random hexamers containing two 3' phosphorothioate linkages were purchased from Integrated DNA Technologies (IDT, Coralville, OH, USA).

*DNA templates*: Double-stranded circular DNA plasmids were purified from *Escherichia coli* cultures using Nucleobond or NucleoSpin columns (Macherey-Nagel, Düren, Germany). Plasmid p3 is a 5 kilobase CMV-GFP expression construct containing a single T7 promoter consensus sequence (5' TAATACGACTCACTATAG 3'); pGEM-7Zf+ (Promega Corporation, Madison, WI, USA, hereinafter referred to as pGEM) is an empty, 3 kilobase standard cloning vector containing a T7 promoter consensus sequence and a SP6 promoter consensus sequence (5' ATTTAGGTGACACTATAG 3') in reverse orientation; pBlueScript II SK+ (Stratagene, La Jolla, CA, USA, hereinafter referred to as pBSK) is a widely used, 3 kb phagemid cloning vector harbouring a T7 promoter and a T3 promoter consensus sequence (5' AATTAACCCTCACTAAAG 3') in reverse orientation. Plasmid p53 is a 3.7 kilobase CMV-GFP mammalian expression vector devoid of any T7, T3, or SP6 promoter sites. Plasmid B327 (3.8 kilobase) was constructed from p53 by inserting BsaI restriction sites and T7 promoter consensus sequences at either side of the CMV-GFP expression cassette in reverse orientation. All plasmids contain a single, unique XbaI recognition sequence for linearization. HeLa genomic DNA (herein referred to as gDNA) and single-stranded, circular DNA from M13mp18 was purchased from NEB (cat. N40065 and N4040S, respectively).

*Standard amplification conditions*: Unless specified otherwise, standard amplification conditions were performed in 0.5 ml or 0.2 ml PCR tubes containing 20 µl reaction volumes as follows. Reactions were prepared in 1x Phi29 buffer B containing 33 mM Tris-acetate (pH 7.9 at 37°C), 66 mM potassium acetate, 10 mM magnesium acetate, 0.1% Tween-20 and 1 mM DTT, or in 1x Phi29 buffer N containing 50 mM Tris-HCl (pH 7.5 at 25°C), 10 mM MgCl₂, 10 mM (NH4)2SO4, and 4 mM DTT. Reaction mixtures were supplemented with 1 mM dNTPs (NEB, N0447), and 0.25 U/µl Phi29 DNA polymerase (Biolab Innovative Research Technologies, EN-20). In all experiments, premixes were used containing all common components to minimize variation between samples. Where appropriate, template DNA, hexamers, ribonucleotides, and/or RNA polymerases were added to premixes or to individual reactions as indicated. All pipetting steps were performed on ice. Reaction mixtures were incubated at 30°C for 16 hours or for the indicated time, followed by heat inactivation at 65°C for 10 min in an PTC-200 Peltier Thermal Cycler DNA Engine (MJ Research), equipped with two 30-well alpha units for 0.5 mL tubes or a 96-well T100 thermo cycler (Bio-Rad) for 0.2 ml tubes.

*Analysis of DNA amplification products*: Following incubation and heat inactivation for the indicated times, reaction mixtures were diluted five times in 5 mM EDTA (pH 8.0) and incubated at 65°C for 30 minutes to solubilize magnesium-pyrophosphate and high molecular weight DNA which often formed a gel-like precipitate due to excessive nucleotide conversion and DNA amplification. Solubilized reaction products were then diluted one time in water to reach an final concentration of 2mM EDTA and stored at -20°C for further analysis.

*Restriction analysis*: For verification of the amplification products, 3 to 5 µl of diluted reaction mixture was digested with 5 units XbaI or BsaI in CutSmart buffer (New England Biolabs, 50 mM Potassium Acetate, 20 mM Tris-acetate, 10 mM Magnesium Acetate, 100 µg/ml BSA, pH 7.9 at 25°C). Following incubation for 1 hour at 37°C, 4pl 6x loading buffer (NEB; Ipswitch, MA, USA) was added and digestion products were analyses on agarose gel.

*Gel electrophoresis*: Samples were loaded on a 1% agarose gel containing ethidium bromide and subjected to electrophoretic analysis in 1x TAE buffer (40 mM Tris, 20 mM acetic acid, 1 mM EDTA). For reference and size estimation of DNA fragments, 1 kb DNA ladder (NEB, N3232) was used. Amplification and/or restriction digestion products were visualized using a UV transilluminator and captured on a ProXima 10 Phi imaging platform.

### Example 2

To investigate whether RNA polymerases could initiate specific priming of DNA amplification by binding to their cognate promoter sequence, different plasmid DNA templates harbouring none, one, or two consensus promoter sequences for different RNA polymerases as indicated in Table 1 were tested in a standard amplification reaction.

Standard amplification reactions were set up in 1x Phi29 buffer B containing 0.25 U/µl Phi29, 1 mM dNTPs, 0.5 mM ribonucleotides GTP/ATP, 10ng template DNA or water, and either 50 µM hexamers (upper left part of Figure 1A), 0.5 U/µl T7 RNA polymerase (upper right part), 0.5 U/µl T3 RNA Polymerase (lower left part), or 0.5 U/µl SP6 RNA Polymerase (lower right part of Figure 1A). Following a 16 hour incubation at 30°C, reaction products were diluted and linearized by XbaI restriction enzyme digestion prior to analysis by gel electrophoresis. Unexpectedly, despite the widely described, high specificity of T7 RNA polymerase for its cognate promoter sequence, T7 RNA polymerases was found to efficiently enable priming of DNA amplification for a DNA template without such a promoter sequence (plasmid p53, lanes 2). Plasmids containing a single T7 promoter sequence (p3, lanes 3) or containing two T7 promoter sequences in reverse orientation (pB327, lanes 4), were equally well amplified in the presence of T7 RNA polymerase, suggesting that priming occurs independently of the T7 promoter sequence. Surprisingly, also T3- and SP6 RNA polymerases, which are also known to be strictly specific for their respective promoter sequences, were found to efficiently enable priming of all used plasmid DNA templates, independent on the presence of their cognate promoter sequences.

This experiment, in addition to other examples described below, thus indicates that RNA polymerases enable primer-less amplification of DNA. Given the simplicity of the method, and the broad availability of the used enzymes and reaction components, the DNA amplification method of the present invention may prove to be a valuable tool in various applications such as for research, diagnostic, and therapeutic purposes.

**Table 1. DNA templates.**

| Lane nr. | Plasmid | Size (kb) | T7 promoter | T3 promoter | SP6 promoter |
|---|---|---|---|---|---|
| 1 | - | | | | |
| 2 | P53 | 3.7 | - | - | - |
| 3 | P3 | 5 | 1 | | |
| 4 | pB327 | 3.8 | 2 | | |
| 5 | pBSK | 3 | 1 | 1 | |
| 6 | pGEM | 3 | 1 | | 1 |

To further analyse and confirm if indeed priming by RNA polymerases occurs independently of their promoter sequences, plasmid pB327 (containing two T7 promoters) was amplified under standard amplification conditions in the presence of either random hexamers, T7 RNA polymerase, T3 RNA polymerase, or SP6 RNA polymerase, in the absence or in the presence of purinergic ribonucleotides. Each reaction contained the following components: 1 mM dNTPs (NEB, N0447) and 0.25 U/µl Phi29 (Blirt, EN20) in 1x Phi29 reaction buffer B. Reactions 2 and 3 (corresponding to the lane numbering in Figure 1B) were supplemented with 50 µM random hexamers, reactions 4 and 7 were supplemented with 0.5 U/µl T7 RNA polymerase, while reactions 5 and 6 were supplemented with 0.5 U/µl T3 RNA polymerase or 0.5 U/µl SP6 RNA polymerase, respectively. Reactions 2 to 5 were further supplemented with ribonucleotides GTP and ATP (0.5 mM each), and lastly, all reactions were provided with 10 ng pB327 template DNA. Reactions were incubated at 30°C for 16h, followed by BsaI restriction analysis and gel electrophoresis. For reference, 200 ng of non-amplified template DNA pB327 was included in the restriction analysis to verify the obtained restriction patterns (lane 8 in Figure 1B).

As depicted, both hexamers and the RNA polymerases enable priming of template DNA amplification, although hexamers do appear to be very effective in priming native plasmid DNA. Although the used template DNA pB327 contains only two promoter consensus sequences for T7 RNA polymerase, also T3 and SP6 RNA polymerase were found to efficiently prime DNA amplification of pB327. If T7 RNA polymerase was expected to bind its cognate promoter sequences and prime DNA amplification from the promoter sites, an exponential amplification would have been expected given the reverse orientation of the T7 promoter sites. Clearly however, the intensity of the amplified products in the T7 RNA polymerase-primed reaction does not exceed those in the T3- or SP6 RNA polymerase reaction (compare lanes 4, 5, and 6), again indicating that priming by RNA polymerases occurs independent of their cognate promoter sequences. Interestingly, in the absence of ribonucleotides, no amplification product was observed in a reaction mixture with T7 RNA polymerase (compare lanes 4 and 7), prompting us to further investigate the dependence on ribonucleotides.

### Example 3

To investigate whether the observed priming of DNA amplification by RNA polymerases is dependent on specific ribonucleotides or concentrations thereof, standard amplification reactions were set up in which only the type of RNA polymerase or the type(s) of ribonucleotides was varied (Figure 2A). Each reaction contained 1 mM dNTPs, 10 ng p53 template DNA, and 0.25 U/µl Phi29 (BLIRT EN20) in 1x Phi29 reaction buffer B. Either T7-, T3-, or SP6- RNA polymerases were added to the reaction mixture at 0.5 U/µl. Ribonucleotides were added to individual reactions as indicated in Figure 2A. To analyze results, 0.5 µl of each reaction product was run on agarose gel. As can be seen in Figure 2A, all three RNA polymerases enable priming of DNA amplification unless no ribonucleotide was present. Interestingly, UTP appears very ineffective for priming by RNA polymerases, while the use of GTP and/or ATP result in the highest yields. It is therefore preferred to include at least one purinergic ribonucleotide in the method of the present invention.

Optimal concentrations of purinergic ribonucleotides for priming of DNA amplification by RNA polymerases were established by titrating a mix of GTP and ATP from 1 mM to 4 µM each (Figure 2B). Each reaction contained 1 mM dNTPs, 10 ng pB327 template DNA, 0.25 U/µl Phi29 (BLIRT, EN20) in 1x Phi29 reaction buffer B, and 0.5 U/µl of either T7-, T3-, or SP6- RNA polymerase. As negative control, water was added instead of ribonucleotides. With GTP/ATP concentrations as low as 15 micromolar, DNA amplification was still readily observed, indicating that ribonucleotides are essential but may be used in the method of the present invention at a very low concentration compared to deoxynucleotides.

Typically, binding of oligonucleotide or random hexamer primers to double-stranded DNA requires an additional denaturation step in which template DNA is denatured by heat or alkali treatment to allow annealing of primers. Such steps require additional equipment and/or buffers, but more importantly, these added steps increase the risk of contamination by exogenous DNA and may severely affect the quality of the template DNA. To establish whether priming by RNA polymerases also requires denaturation of template DNA, pB327 plasmid was mixed with an excess of random hexamers and either left in its native, double-stranded form, or denatured for 5 minutes at 95°C and slowly cooled down to allow annealing of hexamer primers to template DNA. To minimize confounding effects of possible self-priming due to nicks in the template DNA, pB327 plasmid was first treated with T5 exonuclease. To this end 3 µg pB327 was incubated with 30 units T5 exonuclease (NEB, M0363) in CutSmart buffer for 2 hours at 37°C and purified over a NucleoSpin column (Macherey-Nagel, Düren, Germany). Reactions were set up under standard conditions with a final concentration of 0.5 ng/µl template DNA, 50 µM random hexamers, and 0.5 mM GTP/ATP in each reaction. RNA polymerases were added to 0.5 U/µl as indicated above each lane in Figure 3. Separate aliquots of complete reaction mixtures were incubated at 30°C, and amplification reactions were stopped at the indicated timepoints by a 10 min. heat inactivation at 65°C and storage at -20°C until further analysis. Reaction products were digested with XbaI prior to analysis by gel electrophoresis. Digested reaction products migrate at the expected size of linearized pB327 (3.8 kb), and intensities of the bands increases over time. As can be concluded from Figure 3, efficient amplification by random hexamers requires denaturation of template DNA (compare lanes 1 and 5), whereas the addition of RNA polymerases allows priming and amplification of both native and denatured template DNAs. This implicates that the use of RNA polymerases in DNA amplification obviates the need of a denaturation step, thereby making the process truly isothermal.

### Example 4

Transcription activity of DNA dependent RNA polymerases like T7-, T3, and SP6 RNA polymerases is highly specific for their respective promoter sequences, and this binding region is recognized as a double stranded duplex (Golomb et al,, 1977. J Virol 21: 743-752; Stump and Hall, 1993. Nucleic Acids Res 21: 5480-5484; Maslak and Martin, 1993. Biochemistry 32: 4281-4285; Li et al., 1996. Biochemistry 35: 3722-3727). The present invention illustrates that RNA polymerase can also function to prime DNA amplification, and that this unexpectedly occurs in the absence of a promoter sequence. To establish whether priming by RNA polymerases also occurs on single stranded DNA templates, standard amplification reactions were set up using single-stranded M13mp18 phage DNA as template. M13mp18 (NEB, N4040) is a circular, single-stranded DNA molecule of 7.2 kilobase, comprising a single, unique XbaI recognition site, that allows linearization of M13mp18 after replicating to a double-stranded molecule. The M13mp18 sequence (Genbank accession X02513) lacks any T7-, T3, or SP6-promoter sequences. To test DNA amplification, standard reaction conditions were set up using 1 ng/µl single-stranded M13mp18 template DNA and 0.5 mM GTP/ATP in 1x Phi29 buffer (New England Biolabs). Reaction products were digested with XbaI and analyzed by gel electrophoresis. As shown in Figure 4, the digested reaction products migrate at the expected size of linearized M13mp18 (7.2 kb), indicating that single stranded M13mp18 template is faithfully replicated and amplified into double stranded DNA by both hexamer and RNA polymerase priming. The upper band in Figure 4 corresponds to the wells of the agarose gels, most likely representing single-stranded, high molecular weight concatemers of the M13mpl8 template. This example illustrates that RNA polymerases, apart from functioning independently of their promoter sequences, can also efficiently prime replication and amplification of single stranded DNA templates.

### Example 5

Whole genome amplification (WGA) is a powerful technique to amplify small quantities of genomic DNA for further processing, analysis, or sequencing. Most commercially available kits employ either PCR-based methods (e.g. SMARTer^{®} PicoPLEX^{®}, Takara Bio USA, Mountain View, CA; GenomePlex^{®} WGA kits, Sigma-Aldrich, St. Louis, MO) or use multiple displacement amplification (MDA) such as GenomiPhi^{™} (GE Healthcare, Chicago, IL) and REPLI-g (Qiagen, Hilden, Germany). A common feature of these kits is that they rely on the use of random primers or semi-degenerate oligonucleotides. One of the disadvantages of these methods is that the genomic DNA template needs to be denatured in order for the primers to anneal and prime amplification. In cases where only minute amounts or ancient DNA are available (like for instance in forensics or archeology), denaturation of valuable DNA samples may prove detrimental for downstream analysis. Another disadvantage of using random or semi-random primers is biased amplification of template DNA. This may result in loss of information, in sequencing errors due to mis-priming, or in non-specific amplification by self-priming of the random oligonucleotides (see for instance Hansen et al., 2010. Nucleic Acids Res 38: e131; van Gurp et al., 2013. PLoS ONE 8: e85583; Sabina and Leamon, 2015. Methods Mol Biol 1347: 15-41). A new method for genome amplification was recently reported using a primase-polymerase (PrimPol) isolated from *Thermus thermophilus* HB27 (Richer et al., 2016. Nature Comm 7: 13296), which possesses intrinsic primase activity without a need for exogenously added oligonucleotide primers. However, this method still requires denaturation of template DNA prior to amplification, and the used enzyme is not widely available. In order to analyze the ability of RNA polymerase to prime amplification of native, non-denatured genomic DNA, native HeLa genomic DNA was used as template. Amplification was performed under standard amplification conditions in 1x Phi29 buffer (New England Biolabs) containing 0.5 mM GTP/ATP and either 50 µM random hexamers, 0.5 U/µl RNA polymerases as indicated, or a combination of 50 µM random hexamers and 0.5 U/µl or RNA polymerase (Figures 5A and 5B). As template for amplification 1 ng/µl native HeLa genomic DNA (NEB, N4006) was used, and identical reactions were set up with and without template (Figures 5A and 5B). Following amplification, 1 µl of undigested amplification products were analyzed on a 0.7% agarose gel. As shown in Figure 5A, all reactions except the no priming control in lane 1, resulted in amplification products with a major DNA band migrating around 50-70kb, which is regarded as the maximum product length for Phi29-mediated DNA polymerization. Samples containing random hexamers (lanes 2, 6-8) show higher intensities, and appear to contain more amplified DNA.

However, as can be concluded from the no template controls in Figure 5B, most of the products amplified in the presence of random hexamers appear to be artifacts, most likely resulting from self-priming and extension by random hexamers. In contrast, no template control reactions amplified in the presence of only RNA polymerases lack any non-specific amplification products (lanes 3-5). This strongly suggests that priming by RNA polymerases results in specific amplification of native genomic DNA, making it a superior alternative to existing WGA methods.

### Example 6

It was also investigated whether RNA polymerases change the kinetics of random hexamer-primed DNA amplification, or can enhance the product yield. To this end, standard amplification reactions were performed in 1x Phi29 buffer (Biolab Innovative Research Technologies) containing 50 µM hexamers, 0.5mM GTP/ATP, and 10 ng of pB327 plasmid per reaction. The reaction premix was divided in four parts, and supplemented with either water (hexamers only, upper left panel), or with 0.5 U/µl of the indicated RNA polymerases (Figure 6A). At the indicated time points, reactions were stopped, and equal amounts of amplification products were analyzed by XbaI digestion and gel electrophoresis. To quantify the kinetics, band intensities were determined using ImageJ software (Schneider et al., 2012. Nature Methods 9: 671-675) and graphically analyzed in Microsoft Excel (Figure 6B). Clearly, addition of an RNA polymerase dramatically increased the overall yield of the amplification reactions and maximum yields are reached much faster than with hexamers alone. Maximum yield was determined by depletion of dNTPs in the reaction mixture or by inhibitory effects of pyrophosphate that is formed during polymerization of dNTPs, and this status appears to be reached between 8 and 16 hours for T7- and T3 RNA polymerases, and somewhat earlier for SP6 RNA polymerase.

Commercially available isothermal DNA amplification kits typically use random hexamer primers at very high (50 or even 100 µM) concentrations, which represent a huge molar excess over the amplification products. To investigate whether this concentration can be lowered in the presence of an RNA polymerase without loss of yield, a fixed amount of T7 RNA polymerase and variable amounts of random hexamers were tested in standard amplification reactions. To allow random hexamer annealing to template DNA, heat-denatured p53 plasmid DNA was used as template in this experiment. Moreover, this template does not comprise any consensus promoter sequences for T7 RNA polymerase. Following 16 hours of incubation at 30°C, reaction products were analyzed on gel. Unless T7 RNA polymerase was present, no DNA amplification was observed without random hexamers (Fig. 6C; first lanes of the series). Also, the maximum yield in the presence of T7 RNA polymerase appears to be reached at hexamer concentrations of 20 µM, whereas in the absence of RNA polymerase reactions containing even 100 µM have not reached maximum amplification yields. This indicates that addition of an RNA polymerase and ribonucleotides strongly enhances primer-dependent DNA amplification.

### Example 7

To confirm that the methods of the invention also work with similar enzymes from other suppliers and to test the robustness of the invented method, standard reaction conditions were set up using 10 ng of p53 plasmid DNA as template and Phi29 DNA polymerase from three different suppliers. In addition, RNA polymerases from two different suppliers were included and all possible combinations were tested (Figure 7A). Reactions in panel a. comprise 0.25 U/µl Phi29 polymerase from Blirt; reactions in panel b. comprise 0.25 U/µl Phi29 polymerase from New England Biolabs, and reactions in panel c. comprise 0.25 U/µl Phi29 polymerase from Thermo Fisher. All three DNA polymerases were used in combination with the reaction buffer from the corresponding supplier. Reactions products were digested with XbaI and analyzed by gel electrophoresis. As illustrated in Figure 7A, all six tested RNA polymerases enable efficient priming of DNA amplification, comparable to priming by random hexamers (compare lane 2 and lanes 3-8 in each panel). Moreover, Phi29 DNA polymerases from three different suppliers efficiently amplified template DNA (compare panels a-c), indicating that the method of the invention works with a variety of polymerases from different suppliers. Different intensities of amplification products between lanes suggests that certain combinations of an RNA polymerase and a DNA polymerase from the same or different suppliers may work better than others. To further test the robustness of the method, RNA polymerase-primed DNA amplification was performed in two buffers with significantly different compositions; Phi29 buffer N containing 50 mM Tris-HCl (pH 7.5 at 25°C), 10 mM MgCl₂, 10 mM (NH4)2SO4, and 4 mM DTT (denoted "Tris-HCl buffer" in Figure 7B) or in Phi29 buffer B containing 33 mM Tris-acetate (pH 7.9 at 37°C), 66 mM potassium acetate, 10 mM magnesium acetate, 0.1% Tween-20 and 1 mM DTT (denoted "Tris-Acetate buffer" in Figure 7B). All other reaction components were kept identical: 1 mM dNTPs, 0.5 ng/µl p53 plasmid DNA, 0.25 U/µl Phi29 polymerase (Blirt, EN20), 0.5 mM GTP/ATP. Reactions were primed by adding either 50 µM random hexamers (lanes 1 and 5), or 0.5 U/µl T7 RNA polymerase from Thermo Fisher (lanes 2 and 6), or 0.5 U/µl T3 RNA polymerase from Thermo Fisher (lanes 3 and 7), or 0.5 U/µl SP6 RNA polymerase from Thermo Fisher (lanes 4 and 8) and incubated at 30°C for 16 hours to allow DNA amplification. Reaction products were digested with XbaI and analyzed by gel electrophoresis. As depicted in Figure 7B, no significant differences were observed in yield between the different buffer series. The presented examples thus argue for a robust amplification method, not dependent on any specific enzyme suppliers or buffer compositions.

### Example 8.

Therapeutic applications of DNA, such as gene therapy or DNA vaccines, require highly pure and sequence verified DNA preparations devoid of contaminants. Synthetic, cell-free DNA amplification according to methods of the invention may therefore offer a simple and affordable alternative for amplification and production of DNA-based therapeutics. Alternatively, the methods of the invention may be used in the process of DNA sequencing, preferably when limited amounts of template DNA are present. Production of functional DNA was tested by amplifying a GFP expression vector under various conditions, and transfecting digested amplification products into cells for expression analysis. DNA sequences of amplification products were verified by Sanger sequencing.

### Materials and methods

*Amplification*: DNA for transfection studies was produced under standard reaction conditions using 10 ng of p53 as template DNA. Amplification was initiated by adding 50 µM random hexamers either with or without 0.5 mM GTP/ATP (to exclude a possible influence of ribonucleotides in expression studies), or by 0.5 mM GTP/ATP and 0.5 U/µl T7 RNA polymerase, 0.5 U/µl T3 RNA polymerase or 0.5 U/µl SP6 RNA polymerase. Amplification products of p53 plasmid were digested with PvuI (NEB, R3150) for 2h at 37°C. Plasmid p53 contains a single, unique PvuI recognition site in the vector backbone such that digestion with PvuI results in linearized full-length plasmids comprising an intact expression cassette that drives GFP expression. Following digestion, linear DNA fragments were purified using NucleoSpin Gel and PCR Clean-up (Macherey-Nagel, Düren, Germany), eluted in 10 mM Tris-HCl, 0.1 mM EDTA, and DNA concentration was measured on a NanoDrop spectrophotometer (ThermoFisher).

*Cell culture and transfections*: Human embryonal kidney cells (HEK293, ATCC CRL-1573) and mouse melanoma cells (B16F10, ATCC CRL-6475) were maintained under standard tissue culture conditions in IMDM medium (ThermoFisher-Gibco, 21980-032) supplemented with 5% heat-inactivated fetal bovine serum (FBS, Sigma-Aldrich, F0804) and 1% Penicillin-Streptomycin mixture (Lonza, 17-602E). One day before transfections, 20*10E3 HEK293 or 4*10E3 B16-F10 cells in 100 µl IMDM medium per well were seeded in a 96-well flat bottom tissue culture plate. The next day, transfections were carried out in triplo using Saint-DNA transfection reagent (SD-2001-01, Synvolux Products, Leiden, the Netherlands) according to manufacturer's instructions. Briefly, Saint-DNA was calibrated to room temperature and vortexed for 30 sec. For each well, 50 ng of PvuI-digested and purified RCA product was complexed with 1 ul Saint-DNA in phosphate buffered saline (PBS, Lonza, 17-516F) for 5 min at room temperature, in a total volume of 10 µl. Complexes (10 µl) were added to each well and the cells were placed in a CO2 incubator. After 48h, cells were rinsed once with PBS, harvested by trypsin treatment (ThermoFisher-Gibco) and washed twice with FACS buffer comprising PBS and 0.5% bovine serum albumin (BSA, Biowest, P6154). GFP expression was analyzed on a Guava EasyCyte 5HT (Merck-Millipore, Burlington, MA, USA). Data was analyzed with GuavaSoft 3.3 (Merck).

*DNA sequencing*: In order to verify the identity and fidelity of nucleotide incorporation during amplification, sequencing of RCA products was performed using Sanger sequencing. To this end, plasmid pB327 was amplified under standard amplification conditions in the presence of either random hexamers, or an RNA polymerase. Each reaction contained the following components 1 mM dNTPs, 0.5 mM GTP/ATP, 0.25 U/µl Phi29 (Blirt, EN20) and 10 ng pB327 plasmid DNA template in 1x Phi29 reaction buffer N. Priming was initiated by either 50 µM random hexamers, 0.5 U/µl T7 RNA polymerase, 0.5 U/µl T3 RNA polymerase or 0.5 U/µl SP6 RNA polymerase, as indicated in Table 2 below. Reactions were incubated at 30°C for 16h, followed by XbaI digestion and purification using NucleoSpin Gel and PCR Clean-up columns (Macherey-Nagel, Düren, Germany). DNA sequencing was performed by Baseclear B.V. (Leiden, the Netherlands) using a pB327-specific forward sequencing primer and BigDye^{®} Terminator v3.1 Cycle Sequencing (Fisher Scientific, Landsmeer, the Netherlands), on an ABI 3730 Genetic analyzer ((Fisher Scientific, Landsmeer, the Netherlands).

### Results

For comparing the transfection efficiency and expression of reaction products, p53 template DNA was amplified under standard conditions in the presence of random hexamers or RNA polymerases. The p53 plasmid comprises a expression cassette with a CMV promoter, a Green Fluorescent Protein (GFP), and a beta-globin poly-A signal, allowing single cell expression monitoring by flow cytometry.

As illustrated in Figure 8, left panels, all amplification products were efficiently transfected in both HEK293 cells (up to 90%) and in B16F10 cells (up to 70%), without appreciable differences in numbers of transfected cells. Expression levels, as assessed by the level of GFP fluorescence per cell, also did not show any significant differences between the transfected DNA products. This indicates that (1) the inclusion of ribonucleotides, if any, in the amplification reaction does not affect transcription of the amplification products, and (2) that priming of DNA amplification by RNA polymerases according to the method of the present invention delivers pure, and functional DNA suitable for expression in cells and possible therapeutic purposes thereof.

**Table 2. Fidelity of DNA amplification**

| Priming by | Sequence file name | Identity^{a} | Phred score Q15/Q20^{b} |
|---|---|---|---|
| Hexamer | V190491058 | 100% | 525/522 |
| T7 RNAP | V190491059 | 100% | 522/519 |
| T3 RNAP | V190491060 | 100% | 530/528 |
| SP6 RNAP | V190491061 | 100% | 528/525 |

| | | | |
|---|---|---|---|
| ^{a} Identity refers to the base sequence similarity compared to the original template DNA pB327. ^{b} Q15 values represent the number of bases called correctly with a certainty of 96.8% or higher. For Q20 values reliability is 99% or higher. | | | |

Sequencing results (see Table 2) showed that priming by RNA polymerases can faithfully replicate and amplify template DNA, wherein the amplification products are 100% identical in DNA sequence to the original template DNA. This argues that the method of the invention may also be used for DNA sequencing purposes.

### Example 9

For gene therapy purposes, sustained expression and the reproducibility are important factors for success. To investigate and compare *in vivo* gene expression of synthetic linear DNA, mice were injected intradermally on day 0 and 41 with equimolar amounts of either plasmid DNA (pDNA, 10 ug) or linear DNA (InDNA, 5.4 ug) encoding firefly luciferase, or with phosphate buffered saline as control (n=4 per group). Luciferase activity was measured with an IVIS Spectrum *in vivo* imaging system (Perkin Elmer) at indicated time points. Luciferase activity was comparable in mice injected with plasmid DNA or linear DNA with both starting at approximately 2.0*10⁷ photons/sec (p/s) and declining to approximately 1.0*10⁶ p/s after a month (Figure 9). A second injection of DNA resulted in similar levels and kinetics of luciferase activity for both plasmid DNA and linear DNA, indicating that synthetic linear DNA, produced by the method of the present invention is active *in vivo,* and comparable to plasmid DNA.

### Example 10

### Materials and methods

*Mice and tumor cell lines*: C57BL/6 (Jico) mice and B6 Albino mice (strain B6/Rj-Tyrc/c) were purchased from Jackson laboratory (Bar Harbor, ME, USA) and Janvier Labs (Le Genest-Saint-Isle, France), respectively. Mice were housed under FELASA-compliant conditions and all animal experimentations were approved by and according to guidelines of the Dutch Animal Ethical Committee. B16-OVA, a derivative of the B16-F10 melanoma cell line stably transfected with ovalbumin, was maintained in culture medium consisting of IMDM (ThermoFisher-Gibco, Waltham, MA, USA) supplemented with 8% fetal calf serum (Sigma-Aldrich, Zwijndrecht, the Netherlands) in the presence of L-glutamine, penicillin and streptomycin (all from ThermoFisher-Gibco) in a humidified CO2-incubator (37 °C, 5% CO2).

*Linear DNA production for in vivo studies.* Circular plasmid DNA encoding either firefly luciferase or B16-OVA epitopes flanked by BsaI restriction enzyme sites was amplified *in vitro* by the method of the invention. Expression cassettes lacking the plasmid backbone sequences were obtained from concatemeric amplification products by digestion with BsaI (NEB, Ipswich, MA, USA) and ligated to nuclease-resistant hairpin oligos (IDT, Coralville, OH, USA) during 8 cycles of 1h at 37°C and 1h at 16°C, to obtain linear DNA with closed ends (InDNA). T5 exonuclease (NEB, Ipswich, MA, USA) was then added for 16h at 37°C to remove vector backbone and unligated products. Plasmid DNA produced by *E*. *coli* bacterial culture and linear DNA were purified twice with Nucleobond Xtra maxi EF columns (Macherey-Nagel, Düren, Germany) and resuspended in 10% Tris-EDTA buffer.

*In vivo bioluminescence imaging*: On days 0 and 41, B6 Albino mice were injected intradermally at their tail base with equimolar doses of luciferase encoding plasmid DNA or linear DNA. The control group received the same volume of PBS. For *in vivo* bioluminescence imaging, mice were injected subcutaneously with 150mg/kg D-luciferin (Synchem; Cat. Nr. bc219). After 15 minutes, mice were anaesthetized by isoflurane inhalation and the imaging was performed using IVIS Spectrum small animal imager (PerkinElmer). The light signal using an open filter and an automatic acquisition time was quantified at the tailbase of the mice using fixed-sized regions of interest throughout the entire experiment. Image analysis and luminescence quantification was performed using LivingImage software (PerkinElmer).

*Mouse vaccinations, immune responses and tumour challenge*: On day 0, male C57BL/6 mice (3 groups of 8 mice each) were injected intradermally with 30 µl 0,9% NaCl solution containing equimolar amounts (4.3 pmol) of DNA molecules. The first group was vaccinated with 10 µg circular plasmid DNA (3.6 kB) encoding multiple antigens, including an OVA-specific epitope. The second group was vaccinated with 5.4 ug of linear DNA (1.9 kb), amplified from the same plasmid vector as used in the first group but lacking the bacterial backbone sequences, and the third (control) group was left untreated. Two weeks after vaccination, blood was drawn from all mice, treated with erythrocyte lysis buffer and stained with PE-conjugated H2-Kb/SIINFEKL tetramers that were produced at the LUMC tetramer facility, Leiden, the Netherlands, to detect OVA-specific CD8 T cells. Samples were analyzed by flowcytometry on a BD LSRII (Becton Dickinson, San Jose, CA, USA) and FlowJo software (FlowJo LLC). Analysed data were plotted in GraphPad Prism software (San Diego, CA, USA). On day 21 after vaccination, mice were injected subcutaneously with 50,000 B16-OVA cells. Tumour growth was monitored every 3-4 days, and tumour size was calculated as (length x width x width)/2. Mice carrying tumours exceeding 1000 mm3 or with a bleeding ulcer were euthanised by CO2 asphyxiation.

### Results

The methods of the invention are particularly suitable for rapid and inexpensive production of pure DNA, for instance for production of personalized cancer vaccines. To investigate if synthetic linear DNA induces T cell activation and tumor protection, naive 6-8 weeks old C57BL/6 mice received a single intradermal vaccination with equimolar amounts of either plasmid DNA (pDNA, 10 ug) or linear DNA (InDNA, 5.4 ug) encoding OVA epitopes. Mice of a control group were left untreated (n = 8 per group). Tetramer staining and flow cytometry was performed on peripheral blood on day 14 after vaccination to detect induction of OVA-specific T cells. As shown in Figure 10A, vaccination with either plasmid DNA or linear DNA resulted in significant and comparable induction of T cells, specific for the OVA epitope which is encoded within the vaccines. Next, the same mice were challenged with B16-OVA tumor cells (50.000 cells/mouse) 21 days post-vaccination, and tumor growth was followed. Figure 10B shows that all untreated mice (with one exception) died within 1 month due to tumor growth. In contrast, vaccination with plasmid DNA (10 ug) or linear DNA (5.4 ug) resulted in almost full protection against tumor growth. Surviving mice remained tumor-free for the remained of the experiment (well over 100 days).

## Claims

1. A method for amplifying a template DNA molecule, comprising
a) providing a template DNA molecule;
b) providing a promoter-dependent RNA polymerase of the family of single-subunit RNA polymerases, a strand-displacing DNA-dependent DNA polymerase and a combination of ribonucleotides and deoxyribonucleotides;
c) incubating said materials in a suitable buffer and for a suitable amount of time to allow replication and amplification of said template DNA molecule,
wherein priming by said promoter-dependent RNA polymerase occurs independently of a consensus promoter sequence.

2. The method of claim 1, wherein the promoter-dependent RNA polymerase of the family of single-subunit RNA polymerases is selected from the group of T7 RNA polymerase, T3 RNA polymerase, SP6 RNA polymerase, or variants thereof.

3. The method of claim 1 or claim 2, wherein the DNA polymerase is selected from Phi29, Bst, Vent DNA polymerase, or a combination thereof.

4. The method of any one of claims 1-3, wherein said ribonucleotides comprise at most three ribonucleotides, of which at least one ribonucleotide has a purine nucleobase, such as adenine or guanine, or a combination thereof.

5. The method of any one of claims 1-4, wherein replication and amplification further comprises providing at least one oligonucleotide complementary to the template DNA molecule, a mix of random oligonucleotides, or a combination thereof.

6. The method according to any one of claims 1-5, wherein at least one species of the nucleotides or ribonucleotides is modified or labeled, preferably with a detectable label.

7. The method of any one of claims 1-6, wherein the amplified product is detected by fluorescence and/or by chemical means, for example by a biotin- or fluorophore-labeled probe, and/or by amplification in the presence of a fluorophore-conjugated dNTP.

8. Use of a promoter-dependent RNA polymerase of the family of single-subunit RNA polymerases, for initiating priming on a DNA template and, optionally, amplifying the DNA template by a strand-displacing DNA-dependent DNA polymerase in the presence of deoxyribonucleotides, wherein initiating of priming occurs in the presence of at most three ribonucleotides, of which at least one ribonucleotide has a purine nucleobase.

9. Use according to claim 8, wherein the initiating of priming is performed in the presence of 2 or 3 ribonucleotides selected from GTP, ATP, and CTP.

10. Use according to claim 8 or claim 9, for amplifying a single- or double stranded DNA molecule in a linear or circular form, including genomic DNA.

11. Use according to any one of claims 8-10 for the amplification of DNA for therapeutic purposes, including but not limited to gene therapy and vaccines.

12. Use according to any one of claims 8-10, for forensic or diagnostic purposes, for example in genotyping, DNA sequencing, in detecting viral or bacterial DNA, or in detection of a nucleic acid mutation.

13. Use according to any one of claims 8-12, wherein the promoter-dependent RNA polymerase of the family of single-subunit RNA polymerases is selected from the group of T7 RNA polymerase, T3 RNA polymerase, SP6 RNA polymerase, or variants thereof.

14. Use of a kit of parts comprising a promoter-dependent RNA polymerase of the family of single-subunit RNA polymerases, a strand-displacing DNA-dependent DNA polymerase, or a mixture thereof, at most 3 ribonucleotides of which at least one ribonucleotide with has a purine nucleobase, deoxyribonucleotides, and optionally, a part holding a suitable buffer, for amplification of a template DNA molecule.

15. Use according to claim 14, wherein the promoter-dependent RNA polymerase of the family of single-subunit RNA polymerases is selected from the group of T7 RNA polymerase, T3 RNA polymerase, SP6 RNA polymerase, or variants thereof.

## Patentansprüche

1. Verfahren zum Amplifizieren eines Template-DNA-Moleküls, umfassend:
a) Bereitstellen eines Template-DNA-Moleküls;
b) Bereitstellen einer promotorabhängigen RNA-Polymerase der Familie von RNA-Polymerasen mit einer einzigen Untereinheit, einer strangverdrängenden DNA-abhängigen DNA-Polymerase und einer Kombination von Ribonukleotiden und Desoxyribonukleotiden;
c) Inkubieren der Materialien in einem geeigneten Puffer und für einen geeigneten Zeitraum, um Replikation und Amplifikation des Template-DNA-Moleküls zu ermöglichen,
wobei Primen durch die promotorabhängige RNA-Polymerase unabhängig von einer Konsensus-Promotorsequenz stattfindet.

2. Verfahren nach Anspruch 1, wobei die promotorabhängige RNA-Polymerase der Familie von RNA-Polymerasen mit einer einzigen Untereinheit ausgewählt ist aus der Gruppe von T7-RNA-Polymerase, T3-RNA-Polymerase, SP6-RNA-Polymerase oder Varianten davon.

3. Verfahren nach Anspruch 1 oder Anspruch 2, wobei die DNA-Polymerase ausgewählt ist aus Phi29-, Bst-, Vent-DNA-Polymerase oder einer Kombination davon.

4. Verfahren nach einem der Ansprüche 1 bis 3, wobei die Ribonukleotide höchstens drei Ribonukleotide umfassen, von denen mindestens ein Ribonukleotid eine Purin-Nukleobase, wie Adenin oder Guanin oder eine Kombination davon, aufweist.

5. Verfahren nach einem der Ansprüche 1 bis 4, wobei Replikation und Amplifikation weiterhin Bereitstellen mindestens eines Oligonukleotids, komplementär zu dem Template-DNA-Molekül, eine Mischung von zufälligen Oligonukleotiden oder einer Kombination davon umfasst.

6. Verfahren nach einem der Ansprüche 1 bis 5, wobei mindestens eine Spezies der Nukleotide oder Ribonukleotide modifiziert oder markiert ist, vorzugsweise mit einem nachweisbaren Marker.

7. Verfahren nach einem der Ansprüche 1 bis 6, wobei das amplifizierte Produkt durch Fluoreszenz und/oder durch chemische Mittel, beispielsweise durch eine Biotin- oder Fluorophor-markierte Sonde, und/oder durch Amplifikation in Gegenwart eines Fluorophor- konjugierten dNTP nachgewiesen wird.

8. Verwendung einer promotorabhängigen RNA-Polymerase der Familie von RNA-Polymerasen mit einer einzigen Untereinheit zum Initiieren von Primen auf einem DNA-Template und optional Amplifizieren des DNA-Templates durch eine strangverdrängende DNA-abhängige DNA-Polymerase in Gegenwart von Desoxyribonukleotiden, wobei das Initiieren des Primens in Gegenwart von höchstens drei Ribonukleotiden stattfindet, von denen mindestens ein Ribonukleotid eine Purin-Nukleobase aufweist.

9. Verwendung nach Anspruch 8, wobei das Initiieren des Primens in Gegenwart von 2 oder 3 Ribonukleotiden, ausgewählt aus GTP, ATP und CTP durchgeführt wird.

10. Verwendung nach Anspruch 8 oder Anspruch 9 zum Amplifizieren eines einzel- oder doppelsträngigen DNA-Moleküls in einer linearen oder zirkulären Form, einschließlich genomischer DNA.

11. Verwendung nach einem der Ansprüche 8 bis 10 zur Amplifikation von DNA für therapeutische Zwecke, jedoch nicht beschränkt auf Gentherapie und Vakzinen,.

12. Verwendung nach einem der Ansprüche 8 bis 10 für forensische oder diagnostische Zwecke, beispielsweise bei der Genotypisierung, DNA-Sequenzierung, beim Nachweisen von viraler oder bakterieller DNA oder beim Nachweis einer Nukleinsäurenmutation.

13. Verwendung nach einem der Ansprüche 8 bis 12, wobei die promotorabhängige RNA-Polymerase der Familie von RNA-Polymerasen mit einer einzigen Untereinheit ausgewählt ist aus der Gruppe von T7-RNA-Polymerase, T3-RNA-Polymerase, SP6-RNA-Polymerase oder Varianten davon.

14. Verwendung eines Baukastens, umfassend eine promotorabhängige RNA-Polymerase der Familie von RNA-Polymerasen mit einer einzigen Untereinheit, eine strangverdrängende DNA-abhängige DNA-Polymerase oder ein Gemisch davon, höchstens 3 Ribonukleotide, von denen mindestens ein Ribonukleotid eine Purin-Nukleobase aufweist, Desoxyribonukleotide und optional einen Teil, der einen geeigneten Puffer enthält, zur Amplifikation eines Template-DNA-Moleküls.

15. Verwendung nach Anspruch 14, wobei die promotorabhängige RNA-Polymerase der Familie von RNA-Polymerasen mit einer einzigen
Untereinheit ausgewählt ist aus der Gruppe von T7-RNA-Polymerase, T3-RNA-Polymerase, SP6-RNA-Polymerase oder Varianten davon.

## Revendications

1. Procédé pour amplifier une molécule de matrice d'ADN, comprenant
a) la fourniture d'une molécule de matrice d'ADN ;
b) la fourniture d'une ARN polymérase dépendante d'un promoteur de la famille des ARN polymérases à une seule sous-unité, d'une ADN polymérase dépendante de l'ADN à déplacement de brin, et d'une combinaison de ribonucléotides et de désoxyribonucléotides ;
c) l'incubation desdits matériaux dans un tampon convenable et pendant un temps convenable pour permettre la réplication et l'amplification de ladite molécule de matrice d'ADN,
dans lequel l'amorçage par ladite ARN polymérase dépendante d'un promoteur survient indépendamment d'une séquence de promoteur consensus.

2. Procédé selon la revendication 1, dans lequel l'ARN polymérase dépendante d'un promoteur de la famille des ARN polymérases à une seule sous-unité est choisie dans le groupe comprenant l'ARN polymérase de T7, l'ARN polymérase de T3, l'ARN polymérase de SP6, ou leurs variants.

3. Procédé selon la revendication 1 ou 2, dans lequel l'ADN polymérase est choisie parmi les ADN polymérases de Phi29, Bst, Vent, ou leurs combinaisons.

4. Procédé selon l'une quelconque des revendications 1 à 3, dans lequel lesdits ribonucléotides comprennent au plus trois ribonucléotides, parmi lesquels au moins un ribonucléotide a une nucléobase de purine, telle que l'adénine ou la guanine, ou une de leurs combinaisons.

5. Procédé selon l'une quelconque des revendications 1 à 4, dans lequel la réplication et l'amplification comprennent en outre la fourniture d'au moins un oligonucléotide complémentaire de la molécule de matrice d'ADN, d'un mélange d'oligonucléotides aléatoires, ou d'une de leurs combinaisons.

6. Procédé selon l'une quelconque des revendications 1 à 5, dans lequel au moins une espèce des nucléotides ou ribonucléotides est modifiée ou marquée, de préférence avec un marqueur détectable.

7. Procédé selon l'une quelconque des revendications 1 à 6, dans lequel le produit amplifié est détecté par fluorescence et/ou par des moyens chimiques, par exemple par une sonde marquée à la biotine ou par un fluorophore, et/ou par amplification en présence de dNTP conjugué à un fluorophore.

8. Utilisation d'une ARN polymérase dépendante d'un promoteur de la famille des ARN polymérases à une seule sous-unité pour initier un amorçage sur une matrice d'ADN et éventuellement amplifier la matrice d'ADN par une ADN polymérase dépendante de l'ADN à déplacement de brin en présence de désoxyribonucléotides, dans laquelle l'initiation de l'amorçage survient en présence d'au plus trois ribonucléotides, parmi lesquels au moins un ribonucléotide a une nucléobase de purine.

9. Utilisation selon la revendication 8, dans laquelle l'initiation de l'amorçage est effectuée en présence de 2 ou 3 ribonucléotides choisis parmi GTP, ATP et CTP.

10. Utilisation selon la revendication 8 ou la revendication 9 pour amplifier une molécule d'ADN simple ou double brin sous une forme linéaire ou circulaire, y compris un ADN génomique.

11. Utilisation selon l'une quelconque des revendications 8 à 10 pour l'amplification d'ADN à des fins thérapeutiques, y compris, mais sans s'y limiter, la thérapie génique et les vaccins.

12. Utilisation selon l'une quelconque des revendications 8 à 10 à des fins médicolégales ou diagnostiques, par exemple en génotypage, séquençage d'ADN, dans la détection d'ADN viral ou bactérien, ou dans la détection d'une mutation d'acide nucléique.

13. Utilisation selon l'une quelconque des revendications 8 à 12, dans laquelle l'ARN polymérase dépendante d'un promoteur de la famille des ARN polymérases à une seule sous-unité est choisie dans le groupe comprenant l'ARN polymérase de T7, l'ARN polymérase de T3, l'ARN polymérase de SP6, ou leurs variants.

14. Utilisation d'une trousse d'éléments comprenant une ARN polymérase dépendante d'un promoteur de la famille des ARN polymérases à une seule sous-unité, une ADN polymérase dépendante de l'ADN à déplacement de brin, ou un de leurs mélanges, au plus 3 ribonucléotides parmi lesquels au moins un ribonucléotide a une nucléobase de purine, des désoxyribonucléotides, et éventuellement un élément détenant un tampon convenable, pour l'amplification d'une molécule de matrice d'ADN.

15. Utilisation selon la revendication 14, dans laquelle l'ARN polymérase dépendante d'un promoteur de la famille des ARN polymérases à une seule sous-unité est choisie dans le groupe comprenant l'ARN polymérase de T7, l'ARN polymérase de T3, l'ARN polymérase de SP6, ou leurs variants.
